# EUROPEAN PATENT APPLICATION

(11) **EP 4 663 748 A2**
(43) Date of publication of application: **17.12.2025**
(21) Application number: 25202243.9
(22) Date of filing: 18.03.2020
(51) Int. Cl.: C12N 5/00

(54) **METHODS AND SYSTEMS FOR EVALUATION OF CELL SAMPLES**

(30) Priority: 19.03.2019 US 201962820719 P
(62) Divisional of application: 20773626.5
(71) Applicant: Yun, Kyuson, Houston, Texas 77021 (US)
(72) Inventor: Yun, Kyuson, Houston, Texas 77021 (US)
(74) Representative: Richly & Ritschel Patentanwälte PartG mbB

(57) **Abstract**

Described herein are methods and systems for evaluating cell samples, including tissue samples such as cancer tissue. In some cases, evaluating cell samples comprises longitudinal evaluation of one or more candidate molecules. Methods and systems for assessing cellular viability are provided.

## Description

### CROSS-REFERENCE

This application claims the benefit of U.S. Provisional Application No. 62/820,719, filed March 19, 2019, which is incorporated herein by reference in its entirety.

### SUMMARY

Disclosed herein, in some embodiments, is a method of assessing a candidate molecule, comprising: (a) administering the candidate molecule to a cell sample of a tumor tissue derived from a subject; (b) culturing the cell sample in the presence of the candidate molecule for at least 1 day and (c) assessing a response of the cell sample to the candidate molecule. In some embodiments, in (b), the cell sample is cultured for at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, or at least 20 days. In some embodiments, in (b), the cell sample is cultured for 12 days. In some embodiments, in (b), the cell sample is cultured for 16 days. In some embodiments, in (b), the cell sample is cultured for 20 days. In some embodiments, the method further comprises (d) administering an additional candidate molecule to the cell sample and culturing the cell sample for at least 24 hours. In some embodiments, in (d), the cell sample is cultured for at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, or at least 20 days. In some embodiments, in (d), the cell sample is cultured for 4 days. In some embodiments, in (d), the cell sample is cultured for 8 days. In some embodiments, in (d), the cell sample is cultured for 12 days. In some embodiments, the method further comprises (e) assessing a response of the cell sample to the additional candidate molecule. In some embodiments, the method further comprises (d) administering a second dose of the candidate molecule to the cell sample and culturing the cell sample for at least 24 hours. In some embodiments, in (d), the cell sample is cultured for at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days or at least 20 days. In some embodiments, the method further comprises (e) assessing a response of the cell sample to the second dose of the candidate molecule. In some embodiments, in (d), the cell sample is cultured for 4 days. In some embodiments, in (d), the cell sample is cultured for 8 days. In some embodiments, in (d), the cell sample is cultured for 12 days.

In some embodiments, the cell sample comprises a plurality of cells. In some embodiments, the cell sample is an intact tissue. In some embodiments, the cell sample is an organoid. In some embodiments, the cell sample is isolated from the tumor tissue. In some embodiments, the cell sample comprises a cancer cell. In some embodiments, the cell sample comprises a cell from a microenvironment of the tumor tissue. In some embodiments, the cell from the microenvironment of the tumor tissue is an immune cell, a fibroblast, an endothelial cell, a pericyte, an adipocyte, a mesenchymal stem cell, or a bone marrow-derived cell. In some embodiments, the immune cell is a lymphoid cell or a myeloid cell. In some embodiments, the lymphoid cell is a T cell, a B cell, a natural killer cell, a dendritic cell. In some embodiments, the myeloid cell is a basophil, an eosinophil, a mast cell, a neutrophil, a monocyte, an erythrocyte, a macrophage (e.g., a tissue resident and peripherally derived macrophage), a myeloid derived suppressor cell, or a dendritic cell. In some embodiments, the cell sample is cultured in serum-free conditions. In some embodiments, the cell sample is cultured with cytokines or growth factors. In some embodiments, the cell sample is cultured at a liquid-air interface. In some embodiments, the cell sample is submerged in cell culture media. In some embodiments, the response is a viability response, a gene expression response, a protein expression response, a protein modification response, a cell signaling response, a morphology response, or immunephenotype response, or a metabolic response.

In some embodiments, assessing a viability response comprises measuring a metabolic product from the cell sample. In some embodiments, measuring the metabolic product from the cell sample comprises isolating the metabolic product from cell culture media and measuring the metabolic product in the absence of the cell sample. In some embodiments, the metabolic product from the cell sample is a product of a reduction reaction. In some embodiments, the metabolic product from the cell sample is derived from resazurin. In some embodiments, the metabolic product from the cell sample is resorufin. In some embodiments, the metabolic product from the cell sample is derived from a tetrazole. In some embodiments, the tetrazole is selected from the group consisting of 3-(4,5-dimethylthiazol-2-yl)-2,5-diphenyltetrazolium (MTT) or a salt thereof, 2,3-bis-(2-methoxy-4-nitro-5-sulfophenyl)-2H-tetrazolium-5-carboxanilide (XTT) or a salt thereof, and 3-(4,5-dimethylthiazol-2-yl)-5-(3-carboxymethoxyphenyl)-2-(4-sulfophenyl)-2H-tetrazolium (MTS) or a salt thereof. In some embodiments, the metabolic product from the cell sample is a formazan. In some embodiments, the cell sample is from a patient-derived xenograft. In some embodiments, the cell sample is from a primary tumor sample. In some embodiments, the primary tumor sample is a surgical sample obtained from a patient. In some embodiments, the primary tumor sample is a biopsy sample obtained from a patient. In some embodiments, the biopsy sample is obtained by a core needle biopsy.

In some embodiments, the method further comprises recommending treatment of a subject with the candidate molecule. In some embodiments, the method further comprises recommending not treating a subject with the candidate molecule. In some embodiments, the candidate molecule is selected from the group consisting of a biological molecule and a chemical molecule. In some embodiments, the biological molecule is selected from the group consisting of a recombinant protein, an enzyme, an antibody, a growth factor, a receptor, and any portion thereof.

In some embodiments, (a) further comprises providing one or more additional candidate molecules, wherein (c) comprises assessing the response of the cell sample to the candidate molecule and the one or more additional candidate molecules. In some embodiments, the one or more additional candidate molecules are provided simultaneously with the candidate molecule. In some embodiments, the candidate molecule and the one or more additional candidate molecules are provided sequentially. In some embodiments, the method further comprises: (d) administering the candidate molecule to an additional cell sample of the tumor tissue derived from the subject; (e) culturing the additional cell sample in the presence of the candidate molecule for at least 5 days; (f) assessing a response of the additional cell sample to the candidate molecule; and (g) comparing the response of the additional cell sample to the response of the cell sample. In some embodiments, the cell sample is derived from a first location of the tumor tissue and the additional cell sample is derived from a second location of the tumor tissue. In some embodiments, the cell sample and the additional cell sample are obtained from the tumor tissue at different times. In some embodiments, the different times are at least 1, 2, 3, 4, 5, 10, 15, or 20 days apart. In some embodiments, the cell sample and the additional cell sample are obtained from the tumor tissue at substantially the same time. In some embodiments, performing the biological assay on the cell sample comprises permeabilizing the cell sample. In some embodiments, performing the biological assay on the cell sample comprises lysing the cell sample. In some embodiments, the biological assay is selected from the group consisting of flow cytometry, nucleic acid sequencing, polymerase chain reaction, histological analysis, immunohistochemistry, immunofluorescence, enzyme-linked immunosorbent assay (ELISA) analysis, mass spectrometry, and any combination thereof.

Disclosed herein, in some embodiments, is a method of assessing a candidate molecule, comprising: (a) administering the candidate molecule to a cell sample of a tumor tissue derived from a subject; (b) culturing the cell sample of the tumor tissue for at least 1 day; (c) assessing a response of the cell sample of the tumor tissue to the candidate molecule; and (d) repeating (a)-(c). In some embodiments, in (b), the cell sample is cultured for at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, or at least 20 days. In some embodiments, in (b), the cell sample is cultured for 4 days. In some embodiments, (d) comprises repeating (a)-(d) at least 1 time, at least 2 times, at least 3 times, at least 4 times, at least 5 times, or at least 6 times. In some embodiments, (d) comprises repeating (a)-(d) 3 times. In some embodiments, the cell sample comprises a plurality of cells. In some embodiments, the cell sample is an intact tissue. In some embodiments, the cell sample is an organoid. In some embodiments, the cell sample is isolated from the tumor tissue. In some embodiments, the cell sample comprises a cancer cell. In some embodiments, the cell sample comprises a cell from a microenvironment of the tumor tissue. In some embodiments, the cell from the microenvironment of the tumor tissue is an immune cell, a fibroblast, an endothelial cell, a pericyte, an adipocyte, a mesenchymal stem cell, or a bone marrow-derived cell. In some embodiments, the immune cell is a lymphoid cell or a myeloid cell. In some embodiments, the lymphoid cell is a T cell, a B cell, a natural killer cell, a dendritic cell. In some embodiments, the myeloid cell is a basophil, an eosinophil, a mast cell, a neutrophil, a monocyte, a macrophage (e.g., a tissue resident and peripherally derived macrophage), a myeloid derived suppressor cell, an erythrocyte, or a dendritic cell.

In some embodiments, the cell sample is cultured in serum-free conditions. In some embodiments, the cell sample is cultured at a liquid-air interface. In some embodiments, the cell sample is submerged in cell culture media. In some embodiments, the response is a viability response, a gene expression response, a protein expression response, a protein modification response, a cell signaling response, a morphology response, or a metabolic response. In some embodiments, the assessing the response comprises measuring a metabolic product from the cell sample. In some embodiments, measuring the metabolic product from the cell sample comprises isolating the metabolic product from cell culture media and measuring the metabolic product in the absence of the cell sample. In some embodiments, the metabolic product from the cell sample is a product of a reduction reaction. In some embodiments, the metabolic product from the cell sample is derived from resazurin. In some embodiments, the metabolic product from the cell sample is resorufin. In some embodiments, the metabolic product from the cell sample is derived from a tetrazole. In some embodiments, the tetrazole is selected from the group consisting of 3-(4,5-dimethylthiazol-2-yl)-2,5-diphenyltetrazolium (MTT) or a salt thereof, 2,3-bis-(2-methoxy-4-nitro-5-sulfophenyl)-2H-tetrazolium-5-carboxanilide (XTT) or a salt thereof, and 3-(4,5-dimethylthiazol-2-yl)-5-(3-carboxymethoxyphenyl)-2-(4-sulfophenyl)-2H-tetrazolium (MTS) or a salt thereof. In some embodiments, the metabolic product from the cell sample is a formazan.

In some embodiments, the cell sample is from a patient-derived xenograft. In some embodiments, the cell sample is from a primary tumor sample. In some embodiments, the primary tumor sample is a surgical sample obtained from a patient. In some embodiments, the primary tumor sample is a biopsy sample obtained from a patient. In some embodiments, the biopsy sample is obtained by a core needle biopsy. In some embodiments, the method further comprises recommending treatment of a subject with the candidate molecule. In some embodiments, the method further comprises recommending not treating a subject with the candidate molecule. In some embodiments, the candidate molecule is selected from the group consisting of a biological molecule and a chemical molecule. In some embodiments, (a) further comprises providing one or more additional candidate molecules, wherein (c) comprises assessing the response of the cell sample to the candidate molecule and the one or more additional candidate molecules. In some embodiments, the one or more additional candidate molecules are provided simultaneously with the candidate molecule. In some embodiments, the candidate molecule and the one or more additional candidate molecules are provided sequentially. In some embodiments, the method further comprises: (e) administering the candidate molecule to an additional cell sample of the tumor tissue derived from the subject; (f) culturing the additional cell sample of the tumor tissue for at least 1 day; (g) assessing a response of the additional cell sample of the tumor tissue to the candidate molecule; (h) repeating (e) - (g); and (i) comparing the response of the additional cell sample to the response of the cell sample. In some embodiments, the cell sample is derived from a first location of the tumor tissue and the additional cell sample is derived from a second location of the tumor tissue. In some embodiments, the cell sample and the additional cell sample are obtained from the tumor tissue at different times. In some embodiments, the different times are at least 1, 2, 3, 4, or 5 days apart. In some embodiments, the cell sample and the additional cell sample are obtained from the tumor tissue at substantially the same time. In some embodiments, performing the biological assay on the cell sample comprises fixing the cell sample. In some embodiments, performing the biological assay on the cell sample comprises permeabilizing the cell sample. In some embodiments, performing the biological assay on the cell sample comprises lysing the cell sample. In some embodiments, the biological assay is selected from the group consisting of flow cytometry, nucleic acid sequencing, polymerase chain reaction, histological analysis, immunohistochemistry, immunofluorescence, enzyme-linked immunosorbent assay (ELISA) analysis, mass spectrometry, and any combination thereof.

Disclosed herein, in some embodiments, is a method of assessing cellular viability, comprising: (a) culturing a cell sample of a tumor in cell culture media; (b) isolating a metabolic product from the cell culture media; and (c) measuring the metabolic product in the absence of the cell sample, thereby assessing cellular viability of the cell sample. In some embodiments, the cell sample is from a cell line. In some embodiments, the cell sample comprises a plurality of cells. In some embodiments, the cell sample is an intact tissue. In some embodiments, the cell sample is an organoid. In some embodiments, the cell sample is isolated from a tumor sample. In some embodiments, the cell sample comprises a cancer cell. In some embodiments, the cell sample comprises a cell from a microenvironment of a tumor tissue. In some embodiments, the cell from the microenvironment of the tumor tissue is an immune cell, a fibroblast, an endothelial cell, a pericyte, an adipocyte, a mesenchymal stem cell, or a bone marrow-derived cell. In some embodiments, the immune cell is a lymphoid cell or a myeloid cell. In some embodiments, the lymphoid cell is a T cell, a B cell, a natural killer cell, a dendritic cell. In some embodiments, the myeloid cell is a basophil, an eosinophil, a mast cell, a neutrophil, a monocyte, an erythrocyte, a macrophage (e.g., a tissue resident and peripherally derived macrophage), a myeloid derived suppressor cell, or a dendritic cell.

In some embodiments, the cell sample is cultured in serum-free conditions. In some embodiments, the cell sample is cultured at a liquid-air interface. In some embodiments, the cell sample is submerged in cell culture media. In some embodiments, measuring the metabolic product comprises a fluorescence measurement or a chemiluminescence measurement. In some embodiments, the cell sample is cultured for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, or at least 20 days. In some embodiments, the cell sample is cultured for 12 days. In some embodiments, the cell sample is cultured for 16 days. In some embodiments, the cell sample is cultured for 20 days. In some embodiments, the metabolic product from the cell sample is a product of a reduction reaction. In some embodiments, the metabolic product from the cell sample is derived from resazurin. In some embodiments, the metabolic product from the cell sample is resorufin. In some embodiments, the metabolic product from the cell sample is derived from a tetrazole. In some embodiments, the tetrazole is selected from the group consisting of 3-(4,5-dimethylthiazol-2-yl)-2,5-diphenyltetrazolium (MTT) or a salt thereof, 2,3-bis-(2-methoxy-4-nitro-5-sulfophenyl)-2H-tetrazolium-5-carboxanilide (XTT) or a salt thereof, and 3-(4,5-dimethylthiazol-2-yl)-5-(3-carboxymethoxyphenyl)-2-(4-sulfophenyl)-2H-tetrazolium (MTS) or a salt thereof. In some embodiments, the metabolic product from the cell sample is a formazan. In some embodiments, the method further comprises administering a candidate molecule to the cell sample. In some embodiments, the cell sample is from a patient-derived xenograft. In some embodiments, the cell sample is from a primary tumor sample. In some embodiments, the primary tumor sample is a surgical sample obtained from a patient. In some embodiments, the primary tumor sample is a biopsy sample obtained from a patient. In some embodiments, the biopsy sample is obtained by a core needle biopsy. In some embodiments, the method further comprises recommending treatment of a subject with the candidate molecule. In some embodiments, the method further comprises recommending not treating a subject with the candidate molecule.

In some embodiments, the method further comprises: (d) culturing an additional cell sample cell sample in additional cell culture media; (e) isolating a metabolic product from the additional cell culture media; (f) measuring the metabolic product from the additional cell culture media in the absence of the additional cell sample, thereby assessing cellular viability of the additional cell sample; and (g) comparing the cellular viability of the additional cell sample to the cellular viability of the cell sample. In some embodiments, the cell sample is derived from a first location of a tumor tissue and the additional cell sample is derived from a second location of a tumor tissue. In some embodiments, the cell sample and the additional cell sample are obtained from the tumor tissue at different times. In some embodiments, the different times are at least 1, 2, 3, 4, 5, 10, 15, or 20 days apart. In some embodiments, the cell sample and the additional cell sample are obtained from the tumor tissue at substantially the same time. In some embodiments, the tumor tissue is a tissue sample derived from a subject.

Disclosed herein, in some embodiments, is a method of assessing a radiation dose, comprising: (a) administering the radiation dose to a cell sample of a tumor tissue derived from a subject; (b) culturing the cell sample for at least 1 day; and (c) assessing a response of the cell sample to the radiation dose. In some embodiments, in (b), the cell sample is cultured for at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, or at least 19 days, or at least 20 days. In some embodiments, in (b), the cell sample is cultured for 12 days. In some embodiments, in (b), the cell sample is cultured for 16 days. In some embodiments, in (b), the cell sample is cultured for 20 days.

Disclosed herein, in some embodiments, is a method of assessing a tumor treating field, comprising: (a) exposing a cell sample of a tumor tissue derived from a subject to a tumor treating field; (b) culturing the cell sample for at least 1 day; and (c) assessing a response of the cell sample to the tumor treating field. In some embodiments, in (b), the cell sample is cultured for at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, or at least 20 days. In some embodiments, in (b), the cell sample is cultured for 12 days. In some embodiments, in (b), the cell sample is cultured for 16 days. In some embodiments, in (b), the cell sample is cultured for 20 days.

Disclosed herein, in some embodiments, is a method of assessing a radiation dose, comprising: (a) administering the radiation dose to a cell sample of a tumor tissue derived from a subject; (b) culturing the cell sample of the tumor tissue for at least 1 day; (c) assessing a response of the cell sample of the tumor tissue to the radiation dose; and (d) repeating (a)-(c). In some embodiments, in (b), the cell sample is cultured for at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, or at least 20 days. In some embodiments, in (b), the cell sample is cultured for 4 days. In some embodiments, (d) comprises repeating (a)-(d) at least 1 time, at least 2 times, at least 3 times, at least 4 times, at least 5 times, or at least 6 times. In some embodiments, (d) comprises repeating (a)-(c) 3 times.

Disclosed herein, in some embodiments, is a method of assessing a tumor treating field, comprising: (a) exposing a cell sample of a tumor tissue derived from a subject to a tumor treating field; (b) culturing the cell sample of the tumor tissue for at least 1 day; (c) assessing a response of the cell sample of the tumor tissue to the tumor treating field; and (d) repeating (a)-(c). In some embodiments, in (b), the cell sample is cultured for at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, or at least 20 days. In some embodiments, in (b), the cell sample is cultured for 4 days. In some embodiments, (d) comprises repeating (a)-(d) at least 1 time, at least 2 times, at least 3 times, at least 4 times, at least 5 times, or at least 6 times. In some embodiments, (d) comprises repeating (a)-(c) 3 times.

Disclosed herein, in some embodiments, is a method of assessing a tumor treating field, comprising (a) exposing a cell sample of a tumor tissue derived from a subject to a tumor treating field; (b) culturing the cell sample of the tumor tissue for at least 1 day; (c) assessing a response of the cell sample of the tumor tissue to the tumor treating field; and (d) repeating (a)-(c).

Disclosed herein, in some embodiments, is a method of assessing a candidate molecule, comprising (a) administering the candidate molecule to a cell sample of a tumor tissue derived from a subject; (b) culturing the cell sample in the presence of the candidate molecule for at least 12 days; and (c) assessing a response of the cell sample to the candidate molecule.

Disclosed herein is a method of assessing a candidate molecule, comprising (a) administering the candidate molecule to a cell sample of a tumor tissue derived from a subject; (b) culturing the cell sample of the tumor tissue for at least 12 days; (c) assessing a response of the cell sample of the tumor tissue to the candidate molecule; and (d) repeating (a)-(c).

Disclosed herein, in some embodiments is a method of assessing cellular viability, comprising (a) culturing a cell sample of a tumor in cell culture media for at least 4 days; (b) isolating a metabolic product from the cell culture media; and (d) measuring the metabolic product in the absence of the cell sample, thereby assessing cellular viability of the cell sample.

Disclosed herein, in some embodiments, is a method of assessing a candidate molecule, comprising (a) administering the candidate molecule to a cell sample of a tumor tissue derived from a subject; (b) culturing the cell sample of the tumor tissue for at least 4 days; (c) assessing a response of the cell sample of the tumor tissue to the candidate molecule; and (d) repeating (a)-(c) at least 3 times.

Disclosed herein, in some embodiments, is a method of assessing a radiation dose, comprising (a) administering the radiation dose to a cell sample of a tumor tissue derived from a subject; (b) culturing the cell sample for at least 12 days; and (c) assessing a response of the cell sample to the radiation dose.

Disclosed herein, in some embodiments, is a method of assessing a tumor treating field, comprising (a) exposing a cell sample of a tumor tissue derived from a subject to a tumor treating field; (b) culturing the cell sample for at least 12 days; and (c) assessing a response of the cell sample to the tumor treating field.

Disclosed herein, in some embodiments, is a method of assessing a radiation dose, comprising (a) administering the radiation dose to a cell sample of a tumor tissue derived from a subject; (b) culturing the cell sample of the tumor tissue for at least 12 days; (c) assessing a response of the cell sample of the tumor tissue to the radiation dose; and (d) repeating (a)-(c).

Disclosed herein, in some embodiments, is a method of assessing a tumor treating field, comprising (a) exposing a cell sample of a tumor tissue derived from a subject to a tumor treating field; (b) culturing the cell sample of the tumor tissue for at least 12 days; (c) assessing a response of the cell sample of the tumor tissue to the tumor treating field; and (d) repeating (a)-(c).

### BRIEF DESCRIPTION OF THE DRAWINGS

The patent or application file contains at least one drawing executed in color. Copies of this patent or patent application publication with color drawing(s) will be provided by the Office upon request and payment of the necessary fee. The novel features of the disclosure are set forth with particularity in the appended claims. A better understanding of the features and advantages of the present disclosure will be obtained by reference to the following detailed description that sets forth illustrative embodiments, in which the principles of the disclosure are utilized, and the accompanying drawings of which:
FIGs. 1A-1C show viability measurements at 0, 4, 8, and 12 days *in vitro* (div) for breast (FIG. 1A), pancreatic (FIG. 1B), and glioblastoma (FIG. 1C) tumor samples.
FIG. 1D shows histological analysis (H&E staining) of PA0170 pancreatic patient derived xenograft (PDX) tissue after 0, 1, 3, 5, and 13 days in vitro.
FIGs. 1E-1G show H&E staining of human subject tumor tissues from breast cancer (FIG. 1E), lung cancer (FIG. 1F), and kidney cancer (FIG. 1G).
FIGs. 1H and 1I show correlation between viability readings (FIG. 1H) and manual cell counts (FIG. 1I) as measured in the same tumor slices.
FIG. 1J shows viability readings from GBM tissue cultured submerged in media or at an air-media interface, as indicated.
FIG. 2A shows viability readings for BR0851 TNBC PDX tumor cells when treated with Cisplatin or Doxorubicin.
FIG. 2B shows viability readings for BR1126 TNBC PDX tumor cells (FIG. 2B) when treated with Cisplatin or Paclitaxel
FIG. 2C shows immunohistochemical analysis of BR0851 TNBC PDX slices treated with cisplatin or doxocyclin for six days *in vitro.*
FIG. 2D shows viability analysis of SN289 GBM PDX tissue treated with radiation alone, temozolomide (TMZ) plus radiation, or Procarbazine, lomustine and vincristine (PCV) plus radiation at days 0, 4, 8, and 12 of culture.
FIG. 2E shows viability analysis of a colorectal cancer PDX model treated with oxaliplatin, 5-Fluorouracil (5-FU), or the combination (Ox + 5FU) at days 0, 4, and 8 of culture.
FIGs. 3A and 3B show viability analysis of CN0458 colorectal PDX tissue (FIG. 3A) and CNSTG colorectal PDX (FIG. 3B) tissue treated with vehicle, oxaliplatin (OXP), or 5-Fluorouracil (5FU) at the indicated concentrations for the indicated times.
FIGs. 3C and 3D show viability analysis of BR11267 TNBC PDX (FIG. 3A) and BR0851 TNBC PDX (FIG. 3B) cells treated with vehicle or Cisplatin (Cis) at the indicated concentrations for the indicated times.
FIG. 4A shows viability measurements for CN0458 colon PDX tumors treated with one of 119 drugs, as indicated.
FIG. 4B shows viability measurements for the CN0458 colon PDX tumors treated with seven of the 119 drugs shown in FIG. 4A at higher magnification.
FIG. 5A shows the viability response of PDX tumor slices from 4 different patients (CN1571, CN1572, CN1473, and CN1574) to dabrafeninb plus trametinib treatment.
**FIG. 5B** shows treatment response correlation among matching patients, PDX mouse models, and PDX tumor slices to Dabrafenib plus Trametinib treatment.

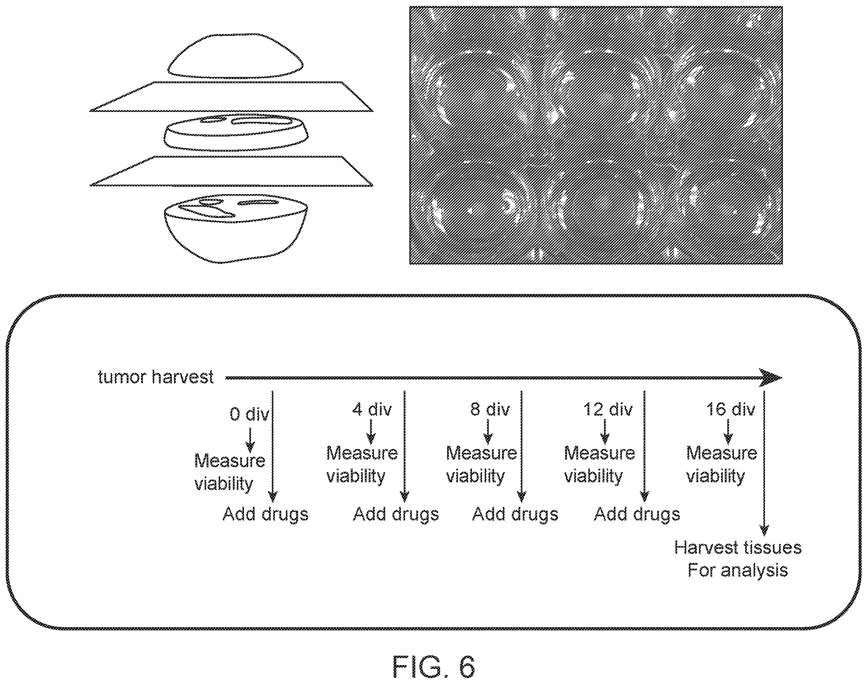

**FIG. 6** shows an example experimental paradigm for measuring viability from a tumor tissue.
**FIG 7** shows an exemplary growth curve and treatment responses using 18gauge biopsy needle cores from MMTV-PyMT breast and CT26 colorectal tumors. MMTV-PyMT biopsy core slices were treated with solvent control (DMSO), cyclophosphamide (CPP, 20µM), doxorubicin (DOX, 100nM), or combination (CPP (20µM) +DOX (100nM)+TAXOL (1nM)). CT26 colorectal tumor biopsy core slices were treated with control (IgG + DMSO), IgG + 5-Fluorouracil (5FU, 1µM), anti-PD1+DMSO, or anti-PD1+5FU+Oxaliplatin (anti-PD1 +5FU (1µM) +OX (1µM)).
**FIG 8** shows emergence of therapy resistance with long-term culture in some tumor tissues. A human mCRC biopsy core treated with encorafenib (E, 100nM, a BRAFV600E inhibitor), cetuximab (C, 10µg/ml, an EGFR inhibitor), binimetinib (B, 100nM, a MEK inhibitor) and combinations show emergence of encorafenib resistance after 16 days in culture, which is suppressed by treatment with a MEK inhibitor binimetinib (compare E vs B, and E+C vs. E+C+B at days 16 and 20).
**FIG 9** shows maintenance of immune cells in tumor slices at 4 days in culture.
**FIG 10** shows use of tumor slices to measure differential immunotherapy responses in human tumors. Needle biopsy cores from two different mCRC patients were treated with control (DMSO), encorafenib (100nM) + cetuximab (E (100nM) +C (10µg/ml)), an immune checkpoint inhibitor nivolumab (N, 10µg/ml), or in combination (E+C+N). Also see Figure 7 for murine breast tumor tissue response to anti-PD1 treatment.

### DETAILED DESCRIPTION

Many cytotoxic and targeted therapies show promising activity in cultured cells, but most fail to show equivalent efficacy in patients, indicating that the predictive power of existing cell culture models is suboptimal. Cell lines (e.g., cancer cell lines) cultured in isolation may fail to fully recapitulate the complexity of human tissue (e.g., tumor tissue) at multiple levels. For example, cancer initiation and progression can be modulated by the tumor microenvironment, including non-cancer cells such as immune cells (e.g., via inflammatory cytokines), stromal cells, etc. Complex model systems, such as patient-derived xenograft (PDX) and other animal models, are often costly and time consuming. Established cell lines, freshly dissociated primary cells, or organoids disrupt native cell:cell communication and extracellular matrices and also lack critical stromal cells, including immune cells.

Disclosed herein, in some embodiments, are methods and systems for evaluating cell samples (e.g., intact cancerous tissue) that recapitulate cellular heterogeneity, retain cell:cell interactions, and are amenable to experimental manipulations in a cost-effective and timely manner. Disclosed herein, in certain embodiments, are methods and systems for evaluating cell samples as intact systems. Disclosed herein, in certain embodiments, are methods and systems for evaluating a cell sample from a subject.

In some embodiments, evaluating a cell sample comprises assessing a candidate molecule (e.g., chemical molecule, biological molecule, etc.) provided to a cell sample for a given duration of time, thereby evaluating the response of the sample to the candidate molecule. In some embodiments, a candidate molecule is administered to a cell sample (e.g., a tumor tissue), the cell sample is cultured in the presence of the candidate molecule for at least 5 days, and a response to the candidate molecule (e.g., cellular viability) is assessed. In some embodiments, the disclosed methods of evaluating a response of a cell sample to a candidate molecule are useful in, for example, identifying the long-term efficacy of one or more compounds in treating a tumor cell from a subject. In some embodiments, the disclosed methods are useful in comparing the long-term efficacy of multiple compounds in treating cells from the same tumor sample. In some embodiments, the disclosed methods are useful for determining changes in a cell state (e.g., maturation of cancer stem cells, repolarization of immune cells, etc.) of a cell sample from a tumor sample. In some embodiments, the disclosed methods are useful for detecting patient-specific response to a candidate molecule or combination of candidate molecules.

In some embodiments, evaluating a cell sample comprises assessing a candidate molecule in a longitudinal manner. In some embodiments, a candidate molecule is administered to a cell sample (e.g., a tumor tissue), the cell sample is cultured in the presence of the candidate molecule for at least 1 day, a response to the candidate molecule (e.g., cellular viability) is assessed, and the process is repeated at least once. In some embodiments, a response of a cell sample to a candidate molecule is accomplished without destruction of the cell sample, thereby enabling continued culturing and assessment of the cell sample. In some embodiments, the disclosed methods of evaluating a response of a cell sample to a candidate molecule are useful in, for example, determining the efficacy of one or more compounds in treating a tumor cell from a subject over time. For example, in some embodiments, the disclosed methods are used to identify one compound as having minimal efficacy against a tumor sample early in treatment and high efficacy later in treatment. In some embodiments, the disclosed methods are useful in comparing the efficacy of multiple compounds in treating cell samples from the same tumor sample over time.

In some embodiments, evaluating a cell sample comprises assessing cellular viability. In some embodiments, disclosed herein are methods of assessing cellular viability. In some embodiments, assessing cellular viability comprises culturing a cell sample in cell culture media, isolating a metabolic product from the cell culture media, and measuring the metabolic product in the absence of the cell sample. In some embodiments, the disclosed methods for assessing cellular viability are useful in, for example, assessing a response of a cell sample (e.g., a tumor cell) to a candidate compound. In some embodiments, measuring a metabolic product from a cell sample in the absence of the cell sample is useful in obtaining measurements with decreased variability as compared with measuring a metabolic product within a cell sample.

### Definitions

As used herein, the term "subject" is used to mean any animal, preferably a mammal, including a human or non-human. The terms patient, subject, and individual are used interchangeably. None of the terms are to be interpreted as requiring the supervision of a medical professional (e.g., a doctor, nurse, physician's assistant, orderly, hospice worker).

The term "about" when referring to a measurable value such as an amount, a temporal duration, and the like, is meant to encompass variations of ±20% or in some instances ±10%, or in some instances ±5%, or in some instances ±1%, or in some instances ±0.1 % from the specified value, as such variations are appropriate to perform the disclosed methods. Further, "about" can mean plus or minus less than 1 or 1, 2, 3, 4, 5, 6, 7, 8, 9, 10, 11, 12, 13, 14, 15, 16, 17, 18, 19, 20, 25, 30, or greater than 30 percent, depending upon the situation and known or knowable by one skilled in the art. About also includes the exact amount. Hence "about 10 kDa" means "about 10 kDa" and also "10 kDa".

### Long term evaluation of a candidate molecule

Disclosed herein, in certain embodiments, are methods for long term evaluation of one or more candidate molecules on a cell sample. In some embodiments, the disclosed methods comprise administering one or more candidate compounds to a cell sample, culturing the cell sample in the presence of the one or more candidate molecules for at least 5 days, and assessing a response of the cell sample to the one of more candidate molecules. In some embodiments, a cell sample comprises a cell derived from a tumor tissue. In some embodiments, a cell sample is cultured for at least 5, at least 6, at least 7, at least 8, at least 9, at least 10, at least 11, at least 12, at least 13, at least 14, at least 15, at least 16, at least 17, at least 18, at least 19, at least 20, at least 25, at least 30 days, or more, prior to assessing a response of the cell sample to the one or more candidate molecules. In some embodiments, a cell sample is cultured for at least 5 days prior to assessing a response. In some embodiments, a cell sample is cultured for at least 6 days prior to assessing a response. In some embodiments, a cell sample is cultured for at least 7 days prior to assessing a response. In some embodiments, a cell sample is cultured for at least 8 days prior to assessing a response. In some embodiments, a cell sample is cultured for at least 10 days prior to assessing a response. In some embodiments, a cell sample is cultured for at least 12 days prior to assessing a response. In some embodiments, a cell sample is cultured for at least 15 days prior to assessing a response. In some embodiments, a cell sample is cultured for at least 16 days prior to assessing a response. In some embodiments, a cell sample is cultured for at least 20 days prior to assessing a response.

In some embodiments, a cell sample comprises a cell. In some embodiments, a cell sample comprises a plurality of cells. In some embodiments, a cell sample is an intact tissue. In some embodiments, a cell sample is an organoid. In some embodiments, a cell sample is isolated from a tumor tissue. In some embodiments, a cell sample comprises a cell from the microenvironment of a tumor tissue. Examples of a cell from the microenvironment of a tumor tissue include, but are not limited to, an immune cell, a fibroblast, an endothelial cell, a pericyte, an adipocyte, a mesenchymal stem cell, and a bone marrow-derived cell. In some embodiments, an immune cell is a lymphoid cell or a myeloid cell. Examples of a lymphoid cell include a T cell, a B cell, a natural killer cell, and a dendritic cell. Examples of a myeloid cell include a macrophage, a tissue-resident monocyte (e.g., a microglial cell), a monocyte-derived suppressor cell, a basophil, an eosinophil, a mast cell, a neutrophil, a monocyte, an erythrocyte, a macrophage (e.g., a tissue resident and peripherally derived macrophage), a myeloid derived suppressor cell, and a dendritic cell. In some embodiments, a cell sample is derived from a patient-derived xenograft sample. In some embodiments, a cell sample is derived from a primary tissue sample (e.g., a primary tumor sample). In some embodiments, a cell sample comprises a cancer cell. In some embodiments, a cell sample comprises a glioblastoma cell. In some embodiments, a cell sample comprises a colon cancer cell. In some embodiments, a cell sample comprises a pancreatic cancer cell. In some embodiments, a cell sample comprises a breast cancer cell. In some embodiments, a cell sample comprises a prostate cancer cell. In some embodiments, a cell sample comprises a kidney cancer cell. In some embodiments, a cell sample comprises a lung cancer cell. In some embodiments, a cell sample comprises a head and neck cancer cell. In some embodiments, a cell sample comprises a skin cancer cell. In some embodiments, a cell sample comprises an ovarian cancer cell. In some embodiments, a cell sample comprises a uterine cancer cell. In some embodiments, a cell sample comprises a sarcoma cell. In some embodiments, a cell sample comprises a liver cancer cell. In some embodiments, a cell sample comprises a gastric cancer cell. In some embodiments, a cell sample comprises a cancer cell that has metastasized to a different organ site (e.g., a breast cancer cell that has metastasized to the lung).

In some embodiments, a cell sample is cultured in conditions sufficient to enable continued viability of the cell sample for at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30 days, or more. In some embodiments, a cell sample is cultured in conditions sufficient to enable continued viability of the cell sample for at least 5 days. In some embodiments, a cell sample is cultured in conditions sufficient to enable continued viability of the cell for at least 6 days. In some embodiments, a cell sample is cultured in conditions sufficient to enable continued viability of the cell for at least 8 days. In some embodiments, a cell sample is cultured in conditions sufficient to enable continued viability of the cell for at least 10 days. In some embodiments, a cell sample is cultured in conditions sufficient to enable continued viability of the cell for at least 12 days. In some embodiments, a cell sample is cultured in conditions sufficient to enable continued viability of the cell for at least 15 days. In some embodiments, a cell is cultured in serum-free medium. In some embodiments, a cell is cultured at a liquid-air interface. In some embodiments, a cell is cultured submerged in cell culture media. In some embodiments, a cell is cultured in a hypoxic environment (e.g., in a hypoxic chamber). In some embodiments, a cell is cultured in reduced pH (e.g., acidic conditions) relative to standard culture conditions. In some embodiments, a cell is cultured in increased pH (e.g., alkaline conditions) relative to standard culture conditions. In some embodiments, a cell is cultured in increased concentrations of amino acids, metabolites, and/or carbon sources relative to standard culture conditions. In some embodiments, a cell is cultured in reduced concentrations of amino acids, metabolites, and/or carbon sources relative to standard culture conditions. In some embodiments, the disclosed methods comprise administering a second dose of one or more candidate molecules to a cell sample. In some embodiments, a cell sample is cultured for at least 24 hours following administering a second dose of one or more candidate molecules. In some embodiments, a cell sample is cultured for at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30 days, or more, following administering a second dose of one or more candidate molecules. In some embodiments, a cell sample is cultured for at least 1 day following administering a second dose of one or more candidate molecules. In some embodiments, a cell sample is cultured for at least 3 days following administering a second dose of one or more candidate molecules. In some embodiments, a cell sample is cultured for at least 5 days following administering a second dose of one or more candidate molecules. In some embodiments, a cell sample is cultured for at least 8 days following administering a second dose of one or more candidate molecules. In some embodiments, a cell sample is cultured for at least 10 days following administering a second dose of one or more candidate molecules. In some embodiments, a cell sample is cultured for at least 12 days following administering a second dose of one or more candidate molecules. In some embodiments, a cell sample is cultured for at least 15 days following administering a second dose of one or more candidate molecules. In some embodiments, a response of a cell sample to the second dose of the one or more candidate molecules is assessed.

In some embodiments, the disclosed methods comprise administering one or more additional candidate molecules to a cell sample. In some embodiments, a cell sample is cultured for at least 24 hours following administering one or more additional candidate molecules. In some embodiments, a cell sample is cultured for at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30 days, or more, following administering one or more additional candidate molecules. In some embodiments, a cell sample is cultured for at least 1 day following administering one or more additional candidate molecules. In some embodiments, a cell sample is cultured for at least 3 days following administering one or more additional candidate molecules. In some embodiments, a cell sample is cultured for at least 5 days following administering one or more additional candidate molecules. In some embodiments, a cell sample is cultured for at least 8 days following administering one or more additional candidate molecules. In some embodiments, a cell sample is cultured for at least 10 days following administering one or more additional candidate molecules. In some embodiments, a cell sample is cultured for at least 12 days following administering one or more additional candidate molecules. In some embodiments, a cell sample is cultured for at least 15 days following administering one or more additional candidate molecules. In some embodiments, a response of a cell sample to the one or more additional candidate molecules is assessed.

In some embodiments, a response of a cell sample to one or more candidate molecules is assessed (e.g., via viability analysis), after which a biological assay (e.g., nucleic acid sequencing, histological analysis, etc) is performed on the cell sample. In some embodiments, assessing a response of a cell sample to one or more candidate molecules does not comprise disrupting (e.g., lysing, permeabilizing, fixing, etc.) the cell samples. In some embodiments, performing a biological assay on a cell sample comprises disrupting (e.g., lysing, permeabilizing, fixing, etc.) the cell samples.

In some embodiments, a response of a cell sample to one or more candidate molecules is compared to a response of an additional cell sample to the one or more candidate molecules. For example, in some embodiments, a candidate molecule is provided to each of two cell samples, each cell sample is cultured for at least 5 days, a response of each cell sample to the candidate molecule is assessed, and the response of each cell sample is compared. In some embodiments, a cell sample and an additional cell sample are obtained from different tumor tissues. In some embodiments, a cell sample and an additional cell sample are obtained from the same tumor tissue. In some embodiments, a cell sample and an additional cell sample are obtained from the same tumor tissue at different times. In some embodiments, a cell sample and an additional cell sample are obtained from the same tumor tissue at substantially the same time. In some embodiments, assessing a response of a candidate molecule to two cell samples obtained from the same tumor tissue is useful in evaluating heterogeneity within a tumor tissue. For example, a cell sample and an additional cell sample may be obtained from different locations of the same tumor tissue at substantially the same time, and a response to a candidate molecule compared from each cell sample, thereby evaluating special heterogeneity in response to the candidate molecule within the tumor tissue. In another example, a cell sample and an additional cell sample may be obtained from the same tumor tissue at different times, and a response to a candidate molecule compared from each cell sample, thereby evaluating heterogeneity in response to the candidate molecule within the tumor tissue over time. In another example, a cell sample from a tumor tissue and a cell sample from a healthy tissue may be obtained from the same subject, and a response to a candidate molecule from each cell sample evaluated.

In some embodiments, the disclosed methods comprise administering one or more candidate molecules to a cell sample in combination with one of more doses of radiation. In some embodiments, the disclosed methods comprise administering one or more candidate molecules to a cell sample in combination with exposure to a tumor treating field. In some embodiments, the disclosed methods comprise administering one or more candidate molecules to a cell sample in combination with immunotherapies.

In some embodiments, the disclosed methods comprise administering one or more doses of radiation to a cell sample, culturing the cell sample for at least 5 days, and assessing a response of the cell sample to the one of more doses of radiation. In some embodiments, a cell sample is be exposed to one or more doses of radiation at least 1, at least 2, at least 3, at least 4, at least 5 times, or more, prior to assessing a response. In some embodiments, the disclosed methods comprise exposing a cell sample to a tumor treating field, culturing the cell sample for at least 5 days, and assessing a response of the cell sample to the tumor treating field.

### Longitudinal evaluation of a candidate molecule

Disclosed herein, in some embodiments, are methods for long term evaluation of one or more candidate molecules on a cell sample. In some embodiments, the disclosed methods comprise administering one or more candidate molecules to a cell sample, culturing the cell sample, assessing a response of the cell sample to the one or more candidate molecules, and repeating the administering, culturing, and assessing with, for example, a second dose of the same one or more candidate molecules or a dose of one or more additional candidate molecules. In some embodiments, a cell sample is cultured for at least 1, at least 2, at least 3, at least 4, at least 5, at least 6, at least 7, at least 8, at least 12, at least 16, at least 24 hours, or more, prior to assessing a response of the cell sample to the one or more candidate molecules. In some embodiments, a cell sample is cultured for at least 12 hours prior to assessing a response. In some embodiments, a cell sample is cultured for at least 24 hours prior to assessing a response.

In some embodiments, a cell sample is cultured for at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30 days, or more, prior to assessing a response of the cell sample to the one or more candidate molecules. In some embodiments, a cell sample is cultured for at least 1 day prior to assessing a response. In some embodiments, a cell sample is cultured for at least 2 days prior to assessing a response. In some embodiments, a cell sample is cultured for at least 3 days prior to assessing a response. In some embodiments, a cell sample is cultured for at least 4 days prior to assessing a response. In some embodiments, a cell sample is cultured for at least 5 days prior to assessing a response. In some embodiments, a cell sample is cultured for at least 10 days prior to assessing a response. In some embodiments, a cell sample is cultured for at least 12 days prior to assessing a response. In some embodiments, a cell sample is cultured for at least 16 days prior to assessing a response. In some embodiments, a cell sample is cultured for at least 20 days prior to assessing a response.

In some embodiments, administering one or more candidate molecules to a cell sample, culturing the cell sample, and assessing a response of the cell sample to the one or more candidate molecules is repeated at least 1, at least 2, at least 3, at least 4, at least 5, at least 6, at least 7, at least 8, at least 9, at least 10 times, or more. In some embodiments, administering one or more candidate molecules to a cell sample, culturing the cell sample, and assessing a response is repeated at least 1 time. In some embodiments, administering one or more candidate molecules to a cell sample, culturing the cell sample, and assessing a response is repeated at least 2 times. In some embodiments, administering one or more candidate molecules to a cell sample, culturing the cell sample, and assessing a response is repeated at least 3 times. In some embodiments, administering one or more candidate molecules to a cell sample, culturing the cell sample, and assessing a response is repeated at least 4 times. In some embodiments, administering one or more candidate molecules to a cell sample, culturing the cell sample, and assessing a response is repeated at least 5 times. In some embodiments, the administering, culturing, and assessing is repeated with an additional dose of the same one or more candidate molecules, for example, to evaluate the longitudinal impact of one or more candidate molecules on a cell sample (e.g., a tumor tissue sample). In some embodiments, the administering, culturing, and assessing is repeated with one or more different candidate molecules, for example, to evaluate the impact of multiple different candidate molecules.

In some embodiments, a cell sample comprises a cell. In some embodiments, a cell sample comprises a plurality of cells. In some embodiments, a cell sample is an intact tissue. In some embodiments, a cell sample is an organoid. In some embodiments, a cell sample is isolated from a tumor tissue. In some embodiments, a cell sample comprises a cell from the microenvironment of a tumor tissue. Examples of a cell from the microenvironment of a tumor tissue include, but are not limited to, an immune cell, a fibroblast, an endothelial cell, a pericyte, an adipocyte, a mesenchymal stem cell, and a bone marrow-derived cell. In some embodiments, an immune cell is a lymphoid cell or a myeloid cell. Examples of a lymphoid cell include a T cell, a B cell, a natural killer cell, and a dendritic cell. Examples of a myeloid cell include a macrophage, a tissue-resident monocyte (e.g., a microglial cell), a monocyte-derived suppressor cell, a basophil, an eosinophil, a mast cell, a neutrophil, a monocyte, an erythrocyte, and a dendritic cell. In some embodiments, a cell sample is derived from a patient-derived xenograft sample. In some embodiments, a cell sample is derived from a primary tissue sample (e.g., a primary tumor sample). In some embodiments, a cell sample comprises a cancer cell. In some embodiments, a cell sample comprises a glioblastoma cell. In some embodiments, a cell sample comprises a colon cancer cell. In some embodiments, a cell sample comprises a pancreatic cancer cell. In some embodiments, a cell sample comprises a breast cancer cell. In some embodiments, a cell sample comprises a prostate cancer cell. In some embodiments, a cell sample comprises a kidney cancer cell. In some embodiments, a cell sample comprises a lung cancer cell. In some embodiments, a cell sample comprises a head and neck cancer cell. In some embodiments, a cell sample comprises a skin cancer cell. In some embodiments, a cell sample comprises an ovarian cancer cell. In some embodiments, a cell sample comprises a uterine cancer cell. In some embodiments, a cell sample comprises a sarcoma cell. In some embodiments, a cell sample comprises a liver cancer cell. In some embodiments, a cell sample comprises a gastric cancer cell. In some embodiments, a cell sample comprises a cancer cell that has metastasized to a different organ site (e.g., a breast cancer cell that has metastasized to the lung).

In some embodiments, a cell sample is cultured in conditions sufficient to enable continued viability of the cell sample for at least 5, at least 6, at least 7, at least 8, at least 9, at least 10, at least 11, at least 12, at least 13, at least 14, at least 15, at least 16, at least 17, at least 18, at least 19, at least 20, at least 25, at least 30 days, or more. In some embodiments, a cell sample is cultured in conditions sufficient to enable continued viability of the cell sample for at least 5 days. In some embodiments, a cell sample is cultured in conditions sufficient to enable continued viability of the cell sample for at least 6 days. In some embodiments, a cell sample is cultured in conditions sufficient to enable continued viability of the cell sample for at least 8 days. In some embodiments, a cell sample is cultured in conditions sufficient to enable continued viability of the cell sample for at least 10 days. In some embodiments, a cell sample is cultured in conditions sufficient to enable continued viability of the cell sample for at least 12 days. In some embodiments, a cell sample is cultured in conditions sufficient to enable continued viability of the cell sample for at least 20 days. In some embodiments, a cell sample is cultured in serum-free medium. In some embodiments, a cell sample is cultured in the presence of cytokines or growth factors. In some embodiments, a cell sample is cultured at a liquid-air interface. In some embodiments, a cell sample is cultured submerged in cell sample culture media. In some embodiments, a cell sample is cultured in a hypoxic environment (e.g., in a hypoxic chamber). In some embodiments, a cell sample is cultured in reduced pH (e.g., acidic conditions) relative to standard culture conditions. In some embodiments, a cell sample is cultured in increased pH (e.g., alkaline conditions) relative to standard culture conditions. In some embodiments, a cell sample is cultured in increased concentrations of amino acids, metabolites, and/or carbon sources relative to standard culture conditions. In some embodiments, a cell sample is cultured in reduced concentrations of amino acids, metabolites, and/or carbon sources relative to standard culture conditions.

In some embodiments, a response of a cell sample to one or more candidate molecules is assessed (e.g., via viability analysis), after which a biological assay (e.g., nucleic acid sequencing, histological analysis, etc) is performed on the cell sample. In some embodiments, a biological assay is performed to evaluate one or more cellular characteristics, for example, cellular morphology, genetic abnormalities, gene expression, protein expression, epigenetic characteristics, protein modification, immunophenotype, metabolite expression, and/or histological characteristics. In some embodiments, a biological assay is an assay for evaluating cellular morphology (e.g., light microscopy, electron microscopy, flow cytometry, etc.). In some embodiments, a biological assay is an assay for evaluating genetic abnormalities (e.g., karyotyping, DNA sequencing, fluorescence in situ hybridization (FISH), etc.). In some embodiments, a biological assay is an assay for evaluating gene expression (e.g., RNA-seq, microarray analysis, northern blot, reverse-transcription polymerase chain reaction (RT-PCR), etc.). In some embodiments, a biological assay is an assay for evaluating protein expression (e.g., immunoblotting, mass spectrometry, flow cytometry, high performance liquid chromatography (HPLC), enzyme-linked immunosorbent assay (ELISA), protein immunoprecipitation, immunofluorescence, immunohistochemistry, etc.). In some embodiments, a biological assay is an assay for evaluating post-translational modification of proteins (e.g., immunoblotting, mass spectrometry, ELISA, immunofluorescence, immunohistochemistry, etc.). In some embodiments, a biological assay is an assay for evaluating epigenetic characteristics (e.g., ATAC-seq, DNase-seq, MNase-seq, bisulfite sequencing, chromatin immunoprecipitation (ChIP), etc.). In some embodiments, a biological assay is an assay for evaluating protein modification (e.g., immunoblotting, mass spectrometry, etc.). In some embodiments, a biological assay is an assay for evaluating metabolite expression (e.g., mass spectrometry, HPLC, NMR, etc.). In some embodiments, a biological assay is an assay for evaluating secreted proteins (e.g., mass spectrometry, cytokine arrays, growth factor arrays, ELISA, bead-based assays, etc). In some embodiments, a biological assay is an assay for evaluating histological characteristics. In some embodiments, assessing a response of a cell sample to one or more candidate molecules does not comprise disrupting (e.g., lysing, permeabilizing, fixing, etc.) the cell samples. In some embodiments, a biological assay is an assay for evaluating changes in immune cell composition, activation states or function. In some embodiments, performing a biological assay on a cell sample comprises disrupting (e.g., lysing, permeabilizing, fixing, etc.) the cell samples.

In some embodiments, a response of a cell sample to one or more candidate molecules is compared to a response of an additional cell sample to the one or more candidate molecules. For example, in some embodiments, a candidate molecule is provided to each of two cell samples, each cell sample is cultured in the presence of the candidate molecule, a response of each cell sample to the candidate molecule is assessed, the process is repeated at least once, and the longitudinal response of each cell sample to the candidate molecule is compared. In some embodiments, a cell sample and an additional cell sample are obtained from different tumor tissues. In some embodiments, a cell sample and an additional cell sample are obtained from the same tumor tissue. In some embodiments, a cell sample and an additional cell sample are obtained from the same tumor tissue at different times. In some embodiments, a cell sample and an additional cell sample are obtained from the same tumor tissue at substantially the same time. In some embodiments, assessing a response of a candidate molecule to two cell samples obtained from the same tumor tissue is useful in evaluating heterogeneity within a tumor tissue. For example, a cell sample and an additional cell sample may be obtained from different locations of the same tumor tissue at substantially the same time, and a response to a candidate molecule compared from each cell sample, thereby evaluating special heterogeneity in response to the candidate molecule within the tumor tissue. In another example, a cell sample and an additional cell sample may be obtained from the same tumor tissue at different times, and a response to a candidate molecule compared from each cell sample, thereby evaluating heterogeneity in response to the candidate molecule within the tumor tissue over time. In another example, a cell sample from a tumor tissue and a cell sample from a healthy tissue may be obtained from the same subject, and a response to a candidate molecule from each cell sample evaluated.

In some embodiments, the disclosed methods comprise administering one or more candidate molecules to a cell sample in combination with one of more doses of radiation. In some embodiments, the disclosed methods comprise administering one or more candidate molecules to a cell sample in combination with exposure to a tumor treating field.

In some embodiments, the disclosed methods comprise administering a dose of radiation to a cell sample, culturing the cell sample, assessing a response of the cell sample to the one of more doses of radiation, and repeating the administering, culturing, and assessing with, for example, a second dose of radiation. In some embodiments, a cell sample is be exposed to one or more doses of radiation at least 1, at least 2, at least 3, at least 4, at least 5 times, or more, prior to assessing a response. In some embodiments, the disclosed methods comprise exposing a cell sample to a tumor treating field, culturing the cell sample, assessing a response of the cell sample to the tumor treating field, and repeating the exposing, culturing, and assessing with, for example, the same tumor treating field or a different tumor treating field.

### Assessing a response

In some embodiments, the disclosed methods comprise assessing a response of a cell sample (e.g., a cell) to one or more candidate molecules. In some embodiments, assessing a response of a cell sample to one or more candidate molecules does not comprise disrupting (e.g., lysing, permeabilizing, fixing, etc.) the cell sample. In some embodiments, a response is a change in one or more cellular conditions in response to exposure to one or more candidate molecules. In some embodiments, a response is, for example, a viability response, a gene expression response, a protein expression response, a protein modification response, a cell signaling response, a morphology response, or a metabolic response. In some embodiments, a response is a viability response. In some embodiments, a viability response is a change in cellular viability in response to exposure to one or more candidate molecules.

In some embodiments, assessing a response of a cell sample to one or more candidate molecules comprises measuring gene expression of a cell sample exposed to the one or more candidate molecules. Methods of measuring gene expression include, but are not limited to, nucleic acid sequencing and microarray analysis. In some embodiments, assessing a response of a cell sample to one or more candidate molecules comprises measuring protein expression of a cell sample exposed to the one or more candidate molecules. Methods of measuring protein expression include, but are not limited to, mass spectrometry, western blotting, immunohistochemistry, immunofluorescence, flow cytometry, mass cytometry and enzyme-linked immunosorbent assay (ELISA). In some embodiments, assessing a response of a cell sample to one or more candidate molecules comprises measuring protein modification of a cell sample exposed to the one or more candidate molecules. Methods of measuring protein modification include, but are not limited to, mass spectrometry, western blotting, and ELISA. In some embodiments, assessing a response of a cell sample to one or more candidate molecules comprises measuring cellular signaling of a cell sample exposed to the one or more candidate molecules. Methods of measuring cellular signaling include, but are not limited to, mass spectrometry, western blotting, nucleic acid sequencing, and microarray analysis. In some embodiments, assessing a response of a cell sample to one or more candidate molecules comprises measuring a morphology change of a cell sample exposed to the one or more candidate molecules. Methods of measuring a morphology change include, but are not limited to, light microscopy and electron microscopy. In some embodiments, assessing a response of a cell sample to one or more candidate molecules comprises measuring metabolite expression of a cell sample exposed to the one or more candidate molecules. Methods of measuring protein modification include, but are not limited to, gas chromatography-mass spectrometry (GC-MS), liquid chromatography-mass spectrometry (LC-MS), and nuclear magnetic resonance (NMR).

In some embodiments, assessing a response of to one or more candidate molecules comprises measuring cellular viability of a cell sample exposed to the one or more candidate molecules. For example, assessing a response of a cell sample to one or more candidate molecules may comprise culturing the cell sample in the presence of the one or more candidate molecules for a given period of time, followed by measuring the cellular viability of the cell sample. In some embodiments, measuring cellular viability of a cell sample comprises measuring a metabolic product from the cell sample. In some embodiments, measuring a metabolic product comprises exciting the metabolic product and measuring the resultant fluorescence (e.g., measuring fluorescence intensity and wavelength). In some embodiments, a metabolic product is measured while inside the cell sample. In some embodiments, a metabolic product from a cell sample is isolated from cell culture media and measured in the absence of the cell sample. In some embodiments, a metabolic product from a cell sample is a result of a reduction reaction. In some embodiments, a metabolic product from a cell sample is derived from a molecule provided to the cell sample. In some embodiments, a metabolic product from a cell sample is derived from resazurin. In some embodiments, a metabolic product is resorufin. In some embodiments, a metabolic product from a cell sample is derived from a tetrazole. Examples of a tetrazole include 3-(4,5-dimethylthiazol-2-yl)-2,5-diphenyltetrazolium (MTT) or a salt thereof, 2,3-bis-(2-methoxy-4-nitro-5-sulfophenyl)-2H-tetrazolium-5-carboxanilide (XTT) or a salt thereof, and 3-(4,5-dimethylthiazol-2-yl)-5-(3-carboxymethoxyphenyl)-2-(4-sulfophenyl)-2H-tetrazolium (MTS) or a salt thereof. In some embodiments, a metabolic product from a cell sample is a formazan.

### Measuring cellular viability

Disclosed herein, in some embodiments, are methods for measuring cellular viability. In some embodiments, the disclosed methods comprise measuring cellular viability in the absence of a cell sample. In some embodiments, the disclosed methods comprise culturing a cell sample (e.g., a cell) in cell culture media, isolating a metabolic product from the cell culture media, and measuring the metabolic product in the absence of the cell sample. In some embodiments, measuring a metabolic product comprises exciting the metabolic product and measuring the resultant fluorescence (e.g., measuring fluorescence intensity and wavelength).

In some embodiments, a cell sample comprises a cell. In some embodiments, a cell sample comprises a plurality of cells. In some embodiments, a cell sample is from a cell line. In some embodiments, a cell sample is an intact tissue. In some embodiments, a cell sample is an organoid. In some embodiments, a cell sample is isolated from a tumor tissue. In some embodiments, a cell sample comprises a cell from the microenvironment of a tumor tissue. Examples of a cell from the microenvironment of a tumor tissue include, but are not limited to, an immune cell, a fibroblast, an endothelial cell, a pericyte, an adipocyte, a mesenchymal stem cell, and a bone marrow-derived cell. In some embodiments, an immune cell is a lymphoid cell or a myeloid cell. Examples of a lymphoid cell include a T cell, a B cell, a natural killer cell, and a dendritic cell. Examples of a myeloid cell include a macrophage, a tissue-resident monocyte (e.g., a microglial cell), a monocyte-derived suppressor cell, a basophil, an eosinophil, a mast cell, a neutrophil, a monocyte, an erythrocyte, and a dendritic cell. In some embodiments, a cell sample is derived from a patient-derived xenograft sample. In some embodiments, a cell sample is derived from a primary tissue sample (e.g., a primary tumor sample). In some embodiments, a cell sample comprises a cancer cell. In some embodiments, a cell sample comprises a glioblastoma cell. In some embodiments, a cell sample comprises a colon cancer cell. In some embodiments, a cell sample comprises a pancreatic cancer cell. In some embodiments, a cell sample comprises a breast cancer cell. In some embodiments, a cell sample comprises a prostate cancer cell. In some embodiments, a cell sample comprises a kidney cancer cell. In some embodiments, a cell sample comprises a lung cancer cell. In some embodiments, a cell sample comprises a head and neck cancer cell. In some embodiments, a cell sample comprises a skin cancer cell. In some embodiments, a cell sample comprises an ovarian cancer cell. In some embodiments, a cell sample comprises a uterine cancer cell. In some embodiments, a cell sample comprises a sarcoma cell. In some embodiments, a cell sample comprises a liver cancer cell. In some embodiments, a cell sample comprises a gastric cancer cell. In some embodiments, a cell sample comprises a cancer cell that has metastasized to a different organ site (e.g., a breast cancer cell that has metastasized to the lung).In some embodiments, a cell sample is cultured in conditions sufficient to enable continued viability of the cell sample for at least 5, at least 6, at least 7, at least 8, at least 9, at least 10, at least 11, at least 12, at least 13, at least 14, at least 15, at least 16, at least 17, at least 18, at least 19, at least 20, at least 25, at least 30 days, or more. In some embodiments, a cell sample is cultured in conditions sufficient to enable continued viability of the cell sample for at least 5 days. In some embodiments, a cell sample is cultured in conditions sufficient to enable continued viability of the cell sample for at least 6 days. In some embodiments, a cell sample is cultured in conditions sufficient to enable continued viability of the cell sample for at least 8 days. In some embodiments, a cell sample is cultured in conditions sufficient to enable continued viability of the cell sample for at least 10 days. In some embodiments, a cell sample is cultured in conditions sufficient to enable continued viability of the cell sample for at least 12 days. In some embodiments, a cell sample is cultured in conditions sufficient to enable continued viability of the cell sample for at least 15 days. In some embodiments, a cell sample is cultured in serum-free medium. In some embodiments, a cell sample is cultured at a liquid-air interface. In some embodiments, a cell sample is cultured submerged in cell culture media.

In some embodiments, a metabolic product from a cell sample is derived from a molecule provided to the cell sample. In some embodiments, a molecule is provided to a cell sample for at least 1, at least 2, at least 3, at least 4, at least 5, at least 6, at least 7, at least 8, at least 12, at least 16, at least 24 hours, or more, prior to measuring a metabolic product from the cell sample. In some embodiments, a molecule is provided to a cell sample for about 1, about 2, about 3, about 4, about 5, about 6, about 7, about 8, about 12, about 16, or about 24 hours, prior to measuring a metabolic product from the cell sample. In some embodiments, a molecule is provided to a cell sample for about 12, about 13, about 14, about 15, about 16, about 17, or about 18 hours prior to measuring a metabolic product from the cell sample. In some embodiments, a molecule is provided to a cell sample for about 12 hours prior to measuring a metabolic product from the cell sample. In some embodiments, a molecule is provided to a cell sample for about 13 hours prior to measuring a metabolic product from the cell sample. In some embodiments, a molecule is provided to a cell sample for about 14 hours prior to measuring a metabolic product from the cell sample. In some embodiments, a molecule is provided to a cell sample for about 15 hours prior to measuring a metabolic product from the cell sample. In some embodiments, a molecule is provided to a cell sample for about 16 hours prior to measuring a metabolic product from the cell sample.

In some embodiments, a metabolic product from a cell sample is a result of a reduction reaction. In some embodiments, a metabolic product from a cell sample is derived from resazurin. In some embodiments, a metabolic product is resorufin. In some embodiments, a metabolic product from a cell sample is derived from a tetrazole. Examples of a tetrazole include 3-(4,5-dimethylthiazol-2-yl)-2,5-diphenyltetrazolium (MTT) or a salt thereof, 2,3-bis-(2-methoxy-4-nitro-5-sulfophenyl)-2H-tetrazolium-5-carboxanilide (XTT) or a salt thereof, and 3-(4,5-dimethylthiazol-2-yl)-5-(3-carboxymethoxyphenyl)-2-(4-sulfophenyl)-2H-tetrazolium (MTS) or a salt thereof. In some embodiments, a metabolic product from a cell sample is a formazan.

In some embodiments, measuring a metabolic product in the absence of a cell sample provides for reduced measurement variability as compared with measuring a metabolic product in the presence of a cell sample (e.g., while inside a cell). For example, in some embodiments, measurements from multiple metabolic products from multiple cells from the same cell sample have less variability when measured in the absence of the cell sample (e.g., isolated from cell culture media) than when measured in the presence of the cell sample (e.g., within a cell).

### Cell samples

In some embodiments, the disclosed methods comprise the use of one or more cell samples. In some embodiments, a cell sample comprises one or more cells. In some embodiments, a cell sample is a cell. In some embodiments, a cell comprises a cancer cell. In some embodiments, a cell sample comprises a cell from a microenvironment of a tumor. Examples of a cell from the microenvironment of a tumor tissue include, but are not limited to, an immune cell, a fibroblast, an endothelial cell, a pericyte, an adipocyte, a mesenchymal stem cell, and a bone marrow-derived cell. In some embodiments, an immune cell is a lymphoid cell or a myeloid cell. Examples of a lymphoid cell include a T cell, a B cell, a natural killer cell, and a dendritic cell. Examples of a myeloid cell include a macrophage, a tissue-resident monocyte (e.g., a microglial cell), a monocyte-derived suppressor cell, a basophil, an eosinophil, a mast cell, a neutrophil, a monocyte, an erythrocyte, and a dendritic cell. In some embodiments, a cell sample is a tissue sample. In some embodiments, a cell sample is derived from a tumor tissue. In some embodiments, a cell sample is derived from a subject (e.g., a tissue from a subject). In some embodiments, a cell sample is derived from a cell line. In some embodiments, a cell sample is an organoid. In some embodiments, a cell sample is derived from an organoid. In some embodiments, a cell sample is derived from a primary tissue (e.g., a primary tumor tissue). In some embodiments, a cell sample is derived from a patient-derived xenograft (PDX). In some embodiments, use of a patient-derived cell sample (e.g., from primary tumor tissue or a patient derived xenograft) allows for the detection of patient-specific response to a candidate molecule. In some embodiments, a cell sample comprises a single type of cell (e.g., cancer cells). In some embodiments, a cell sample comprises multiple types of cells, (e.g., cancer cells and cells from a microenvironment of a tumor). In some embodiments, the cell sample is from a primary tumor sample. In some embodiments, the method of claim 1, wherein the cell sample is from a metastatic tumor sample.

In some embodiments, the primary tumor sample is a surgical sample from a subject. In some embodiments, the primary tumor sample is derived from a patient biopsy (e.g., a removal of tissue from any part of a patient's body, such as a core biopsy, an excisional biopsy, an incisional biopsy, or a punch biopsy). In some embodiments, the biopsy is a core needle biopsy (e.g., a biopsy where a hollow needle is inserted into a tumor to remove a tissue sample from the tumor). In some embodiments, the needle for obtaining the core biopsy is a 14, 15, 16, 17, 18, 19, 20, 21 or 22 gauge needle. In some embodiments, the needle is an 18 gauge needle.

In some embodiments, multiple cell samples are obtained via a core needle biopsy from different anatomical locations on the same tumor. In some embodiments, the multiple samples are obtained at the same point in time. In some embodiments, said samples are obtained at different points in time. For example, in some embodiments, the disclosed methods are used to identify one compound as having minimal efficacy against a tumor sample early in treatment and high efficacy later in treatment.

### Candidate molecules

In some embodiments, the disclosed methods comprise assessing one or more candidate molecules. In some embodiments, a candidate molecule is a therapeutic. In some embodiments, a therapeutic is a cancer therapeutic. In some embodiments, a candidate molecule is a compound suspected of being a therapeutic. In some embodiments, a candidate molecule is not a therapeutic. In some embodiments, a candidate molecule is a molecule suspected of being capable of eliciting a response from a cell sample.

In some embodiments, a candidate molecule is a chemical molecule. In some embodiments, a candidate molecule is a small molecule. In some embodiments, a small molecule is at most 900, at most 800, at most 700, at most 600, at most 500, at most 400, at most 300, at most 200, at most 100 Daltons, or less, in size. In some embodiments, a candidate molecule is a biological molecule. Examples of a biological molecule include, but are not limited to, a recombinant protein, an enzyme, an antibody, an engineered antibody, an engineered antibody-drug chimera, a growth factor, a cytokine, a chemokine, a receptor, and any portion thereof.

### Biological assays

In some embodiments, the disclosed methods comprise performing one or more biological assays on a cell sample. In some embodiments, a biological assay is performed on a cell sample following assessing a response of a cell sample to one or more candidate molecules. For example, a candidate molecule is provided to a cell sample, the cell sample is cultured in the presence of the candidate molecule for at least 5 days, a viability response of the cell sample is assessed, and a biological assay is subsequently performed. In some embodiments, performing a biological assay on a cell sample comprises disrupting (e.g., lysing, permeabilizing, fixing, etc.) a cell of the cell sample. In some embodiments, a biological assay is performed on a cell sample subsequent to long term evaluation of a candidate molecule. In some embodiments, a biological assay is performed on a cell sample subsequent to longitudinal evaluation of a candidate molecule.

In some embodiments, a biological assay comprises fixing a cell. In some embodiments, a biological assay comprises permeabilizing a cell. In some embodiments, a biological assay comprises lysing a cell. In some embodiments, a biological assay is performed to evaluate one or more cellular characteristics, for example, cellular morphology, genetic abnormalities, gene expression, protein expression, epigenetic characteristics, protein modification, metabolite expression, and/or histological characteristics. In some embodiments, a biological assay is an assay for evaluating cellular morphology (e.g., light microscopy, electron microscopy, flow cytometry, etc.). In some embodiments, a biological assay is an assay for evaluating genetic abnormalities (e.g., karyotyping, DNA sequencing, fluorescence in situ hybridization (FISH), etc.). In some embodiments, a biological assay is an assay for evaluating gene expression (e.g., RNA-seq, microarray analysis, northern blot, reverse-transcription polymerase chain reaction (RT-PCR), etc.). In some embodiments, a biological assay is an assay for evaluating protein expression (e.g., immunoblotting, mass spectrometry, flow cytometry, high performance liquid chromatography (HPLC), enzyme-linked immunosorbent assay (ELISA), protein immunoprecipitation, immunofluorescence, immunohistochemistry, etc.). In some embodiments, a biological assay is an assay for evaluating epigenetic characteristics (e.g., ATAC-seq, DNase-seq, MNase-seq, bisulfite sequencing, chromatin immunoprecipitation (ChIP), etc.). In some embodiments, a biological assay is an assay for evaluating protein modification (e.g., immunoblotting, mass spectrometry, etc.). In some embodiments, a biological assay is an assay for evaluating metabolite expression (e.g., mass spectrometry, HPLC, etc.). In some embodiments, a biological assay is an assay for evaluating histological characteristics. In some embodiments, a biological assay is an assay for evaluating secreted molecules.

### EXAMPLES

### Example 1: Long-term culture and longitudinal monitoring of PDX tumor slice

A slice culture system that enables continued growth of intact tumor tissues for >20 days *ex vivo* was developed. 4 parameters were tested in development of the optimal culture conditions, including 5 different culture medium conditions, 4 different viability assays, 3 different culture conditions, and 5 different tumor sizes. These parameters and the final culture conditions are shown in Table 1.

**Table 1**

| **Parameter** | **Variable** | **Final culture condition** |
|---|---|---|
| Medium | 1. TSC medium without phenol red | TSC medium with phenol red |
| | 2. TSC medium with phenol red | |
| | 3. hNSC | |
| | 4. DMEM/F-12+1%FBS | |
| | 5. MammoCult^{™} | |
| Viability assays | 1. Cell-Titer Blue assay (Promega) | Cell-Titer Blue assay |
| | 2. WST-1 (Sigma Aldrich) | |
| | 3. MTS (abcam) | |
| | 4. Cell-Titer Fluor assay (Promega) | |
| Culture condition | 1. Membrane | Membrane |
| | 2. Insert | |
| | 3. Submersion | |
| Tumor sizes | 1. 2mm | 18g biopsy needle core and 3mm diameter 200-350µm thick |
| | 2. 3mm | |
| | 3. 4mm | |
| | 4. 5mm | |
| | 5. 18g biopsy needle core | |

Culture conditions consisted of generating defined-size tumor slices (3mm diameter, 250-350 µM thick) that were cultured at liquid-air interface in serum-free culture medium.

In these conditions, tumor tissues originating from multiple organ sites (e.g., brain, breast, colon, pancreas) were maintained with continued growth for up to 16 days *ex vivo.*

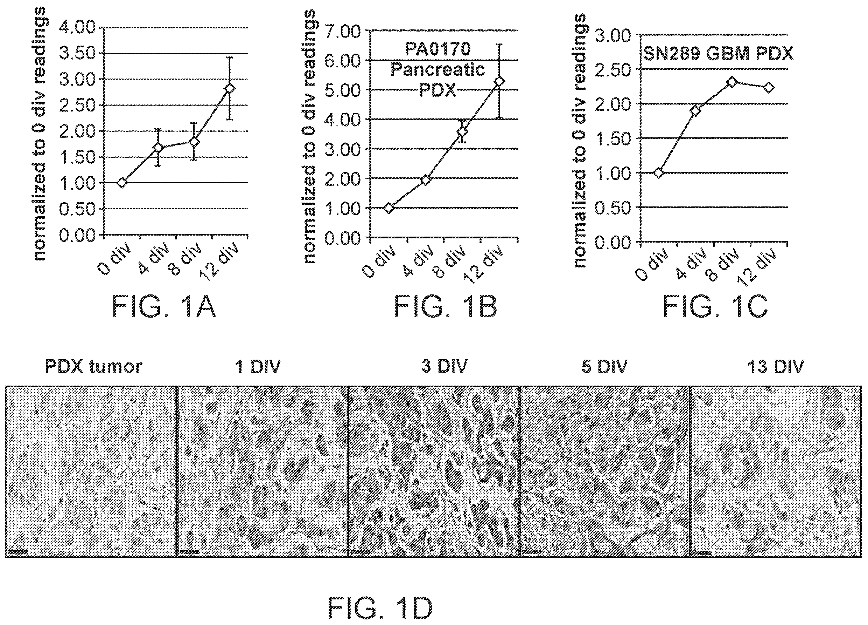

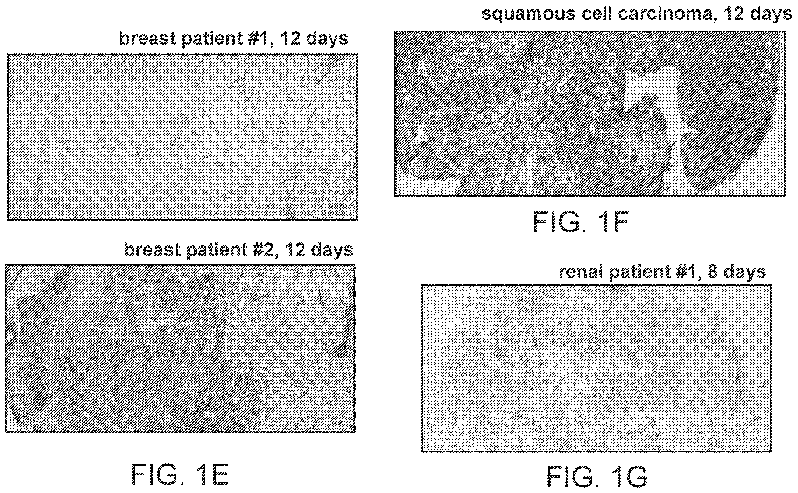

Growth of each sample was measured longitudinally using a fluorescent metabolic viability assay, Cell-Titer Blue (CTB) allowing multiple real-time measurements from the same tissue before and after different perturbations. FIGs. 1A-1C show viability measurements at 0, 4, 8, and 12 days *in vitro* (div) for breast (FIG. 1A), pancreatic (FIG. 1B), and glioblastoma (FIG. 1C) tumor samples. These results demonstrate viability and continued proliferation of cultured tissue under these conditions. FIG. 1D shows histological analysis (H&E staining) of PA0170 pancreatic cells after 0, 1, 3, 5, and 13 days *in vitro.* FIGs. 1E-1G show H&E staining of human patient tumors from breast cancer (FIG. 1E), lung cancer (FIG. 1F), and kidney cancer (FIG. 1G) after the indicated number of days *in vitro.* These results show that tumor tissues from different organ sites retain original histological features even after 13 days in culture.

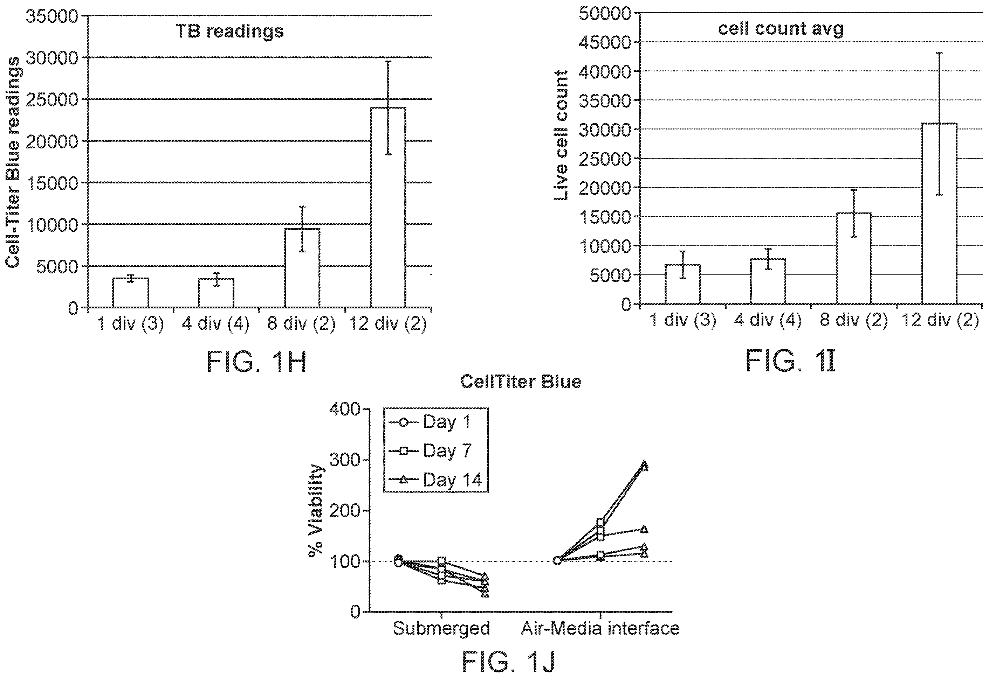

To validate that viability measurements accurately reflect changes in tumor cell numbers, viability readings were compared to actual cell counts from the same tissue. Using multiple different PDX models, viability was measured. Live cells were manually counted on the same tissue immediately after taking the viability reading. The tissues were dissociated and live cells counted from each well by Trypan blue exclusion using a hemocytometer. FIGs. 1H and 1I show correlation between cell count numbers and viability readings in SN211 GBM PDX tissue. FIG. 1J shows viability measurements from GBM tissue cultured in submerged conditions (left) or at an air-media interface (right). Measurements were taken at day 1, 7, or 14, as indicated. These data indicate significantly improved viability and proliferation for tissue cultured at an air-media interface compared with tissue cultured in submerged conditions.

### Materials and Methods

### Patient Derived Xenografts

Early passage (P0-P1) patient derived xenografts (PDX) were obtained from Jax-West and expanded in 2-3 month old female NSG (NOD.Cg-Prkdescid Il2rgtm1Wjl/SzJ; strain: 005557) or NSG-Hprt (NOD.Cg-Prkdcscid Hprtem1Mvw Il2rgtm1Wjl/MvwJ; strain: 026222) or NGS-EGFP (NOD.Cg-Prkdcscid I12rgtm1Wjl Tg(CAG-EGFP)1Osb/SzJ; strain: 021937) mice. 2 triple negative breast cancer (TNBC), 5 Glioblastoma Multiforme (GBM), 3 Pancreatic cancer and 3 Colon cancer samples were tested. GBM and TNBC models were orthotopically expanded by injecting them into brain or 4th mammary fat pad, respectively. Pancreatic and Colon tumor models were injected and propagated subcutaneously.

### Tumor Tissue Harvest and Organotypic Slice Culture

Tumor tissues were harvested when tumors reach 500-1000 mm³. Samples were embedded in 4% low-melt agarose (Bio-Rad^{®}) in KREBs buffer or phosphate buffered saline (PBS) and precision cut into 250-350µM thick slices using a Vibratome VT1200S (Leica Microsystems). Slicing speed and amplitude were adjusted depending on the density and integrity of the tumor tissue. 1, 2 or 3 mm diameter tissues were generated from tumor containing regions using a biopsy needle, avoiding necrotic regions. Each slice punch was cultured on thin, porous membranes (Nucleopore^{™} Track-Etched - Whatman^{®}) in 12-well or 48-well culture dishes with 1 ml or 0.2ml of serum free slice culture medium (slice medium: DMEM/F-12 (Hyclone^{®}), B27 supplement (Gibco^{®}), 100 U/mL penicillin (Gibco^{®}), and 100 µg/mL streptomycin (Gibco^{®})).

### Viability assay and histological analysis

5 µl of Cell-Titer Blue (CTB) (Promega^{®}) substrate was added to 1 ml of culture medium and incubated at 37°C for 15 or 24 hr. 100 µl of media was removed from each sample culture and fluorescence was measured (Ex560/Em590) using Enspire^{®} multimode plate reader (Perkin Elmer^{®}). Background fluorescence of the culture medium was subtracted from each sample reading at each day. Immediately following the CTB reading, media was changed and drugs were added to fresh media to continue culture. At desired time points, additional viability measurements were made to collect longitudinal data and conditioned media is collected and analyzed. After the final time point, tissues were fixed in formalin and embedded in paraffin or OCT for histological analyses or lysed in RIPA buffer or Trizol and conditioned media is frozen. To account for variability in tissue composition of each slice punch, viability readings of each sample was normalized to the respective CTB reading immediately after slicing (0 days ex vivo: 1 dex CTB reading) and relative fold changes in CTB readings at each time point were analyzed.

### Example 2: Testing drug responses using long-term PDX slice cultures

13 drugs were tested either alone or in combination with radiation in 12 different PDX models from the brain, breast, colon, and pancreas (Table 2). Combinations tested included Procarbazine, Lomustine, and Vincristine ("PCV") and 5FU, Leucovorin, Irinotecan, and OXP ("FLCO").

**Table 2**

| **Tumor type** | **Models** | **Treatments tested** |
|---|---|---|
| GBM | 1. SN291 | 1. TMZ + radiation |
| | 2. SN207 | |
| | 3. SN289 | 2. PCV+radiation |
| | 4. SN211 | |
| | 5. SN288 | |
| TNBC | 1. BR1126 | 1. Cis |
| | | 2. Taxol |
| | 2. BR0851 | 3. Dox |
| | | 4. D-Taxol |
| Colon | 1. CN458 | 1. 5FU |
| | 2. CN1572 | 2. OXP |
| | 3. CNSTG | |
| Pancreatic | 1. PA0170 | 1. Gem |
| | 2. PA0172 | 2. Gem+FLCO |
| | 3. PA209 | |

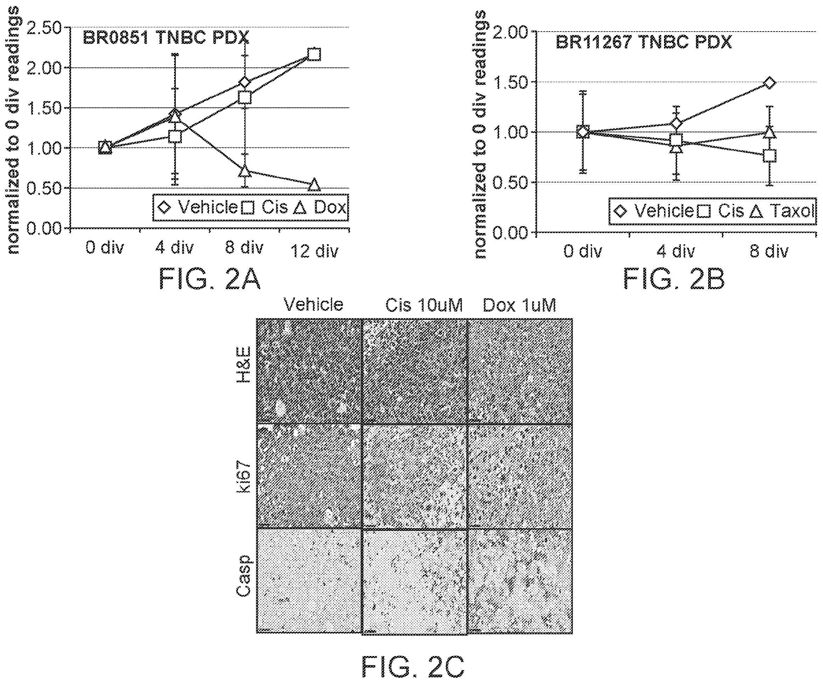

FIGs. 2A and 2B show examples from two triple negative breast cancer (TNBC) PDXs that are representative of observed responses. Two independent TNBC PDX slices were treated with standard care chemotherapy, Cisplatin (Cis), Doxorubicin (Dox) or Paclitaxel (Taxol). Results were observed via viability measurement as described in Example 1. Viability assay readings indicate that BR0851 TNBC PDX tumor is insensitive to Cisplatin treatment but sensitive to Dox treatment, as demonstrated by increased viability readings in vehicle and Cisplatin treated slices and decreased viability readings in Dox treated slices at 12 days *ex vivo* ("div"), compared to 0div (FIG. 2A). On the other hand, BR1126 TNBC PDX tumor is sensitive to both Cis and Taxol treatments (FIG. 2B).

Immunohistochemical analyses of BR0851 TNBC PDX slices at 13div show that overall cell numbers are similar in vehicle and Cis treated tissues while Dox treatment tissues have overall reduction in cell numbers, increase in pyknotic and abnormal nuclei, and increase in necrotic regions compared with vehicle or Cisplatin treated tissues (FIG. 2C). Hence, immunochemical analysis is consistent with CTB readings, i.e. Dox treatment results in a complete response by 13div. In contrast, Cisplatin treated tissues continued to grow and display a progressive disease profile even after long-term exposure to the drug.

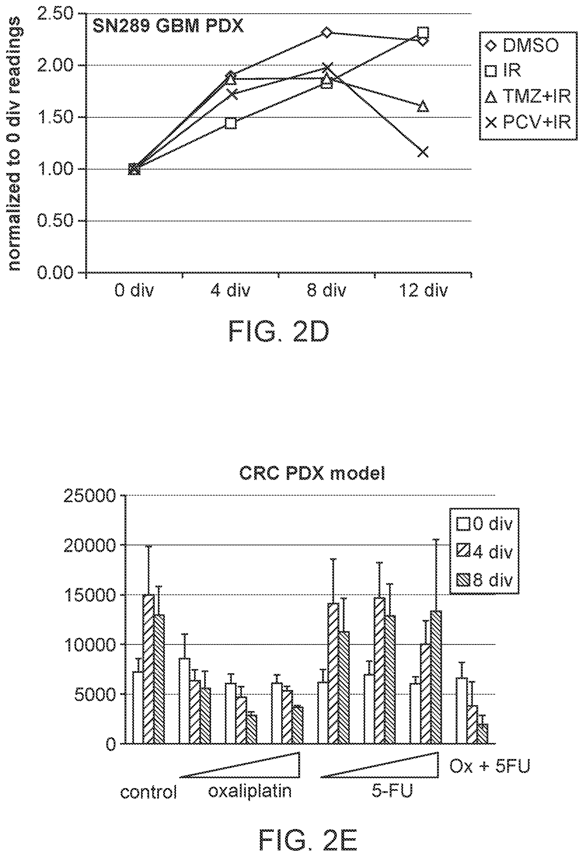

Using a PDX GBM model (SN289), the two treatment regimens were compared side by side on the same tumor. From a single PDX tumor, multiple slice punches were generated and subjected to different treatments in multiple replicates. Radiation treatment by itself (IR) suppressed growth initially but not in long term cultures, whereas TMZ in combination with radiation resulted in reduced viability from 4div to 13 div. (FIG. 2D). PCV in combination with radiation also showed a similar response, resulting in a stable disease profile by 13div (FIG. 2D).

Current standard of care for metastatic colorectal cancer (mCRC) includes oxaliplatin plus 5-fluorouracil (5-FU) treatment. FIG. 2E shows viability measurements observed as described in Example 1 from cells treated with oxaliplatin (0.1 µM, 1 µM, and 10 µM) as indicated from left to right), 5-FU (1 µM, 10 µM, and 100 µM as indicated from left to right), or the combination for the indicated days *in vitro* (div). This data demonstrates a dose and time-dependent response of mCRC to oxaliplatin. In contrast, 5-FU treatment shows no reduction in cell viability, even at the highest dose. These results indicate that such tissue culture and response measurement can be used to eliminate ineffective chemotherapies from combination treatments in individual patients.

### Materials and Methods

### Histology analysis and Immmunohistochemistry

Formalin-fixed, paraffin- or OCT- embedded tissue sections were stained with Hematoxylin and Eosin (H&E) for histological assessment. Specific markers of proliferation (KI67: Abcam^{®}, ab15580, 1:300), and apoptosis (cleaved-caspase 3: Cell Signaling^{®}, #9661, 1:300) were analyzed using standard Immunohistochemical analysis protocol. In addition, it was also confirmed that PDX tumors are of human origin by using a human specific antibody (HuNu: EMD Millipore^{®}, MAB1281 (clone 235-1), 1:300)

### Example 3: Ex vivo slice cultures to predict cross-model and inter-patient differences in drug sensitivity

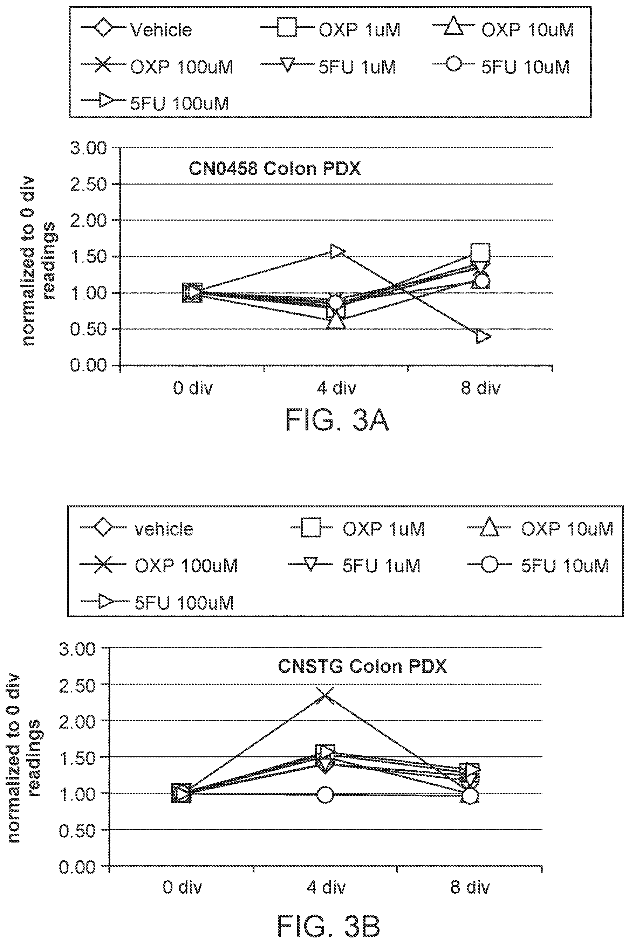

Inter- and intra- tumoral heterogeneity play a crucial role in drug response and emergence of drug resistance. Just as different patients respond to same drugs differently, different PDX models are anticipated to show differential sensitivity to the same drug. To test whether a slice culture system can report differential drug sensitivities of different PDX models, treatment response of independent tumors of the same clinical type were compared. For example, two different colon PDX tumors were treated with standard care agents Oxaliplatin (OXP) and 5-Fluorouracil (5FU), and both colon tumor models responded differently to OXP and 5FU (FIGs. 3A and 3B). Based on viability readings, it is evident 100uM OXP is more effective in suppressing the viability of CNSTG colon tumor slices compared with CN0458 colon tumor slices, whereas 100uM 5FU is more effective in suppressing the viability of CN0458 tumor slices compared with CNSTG colon tumor slices (FIGs. 3A and 3B).

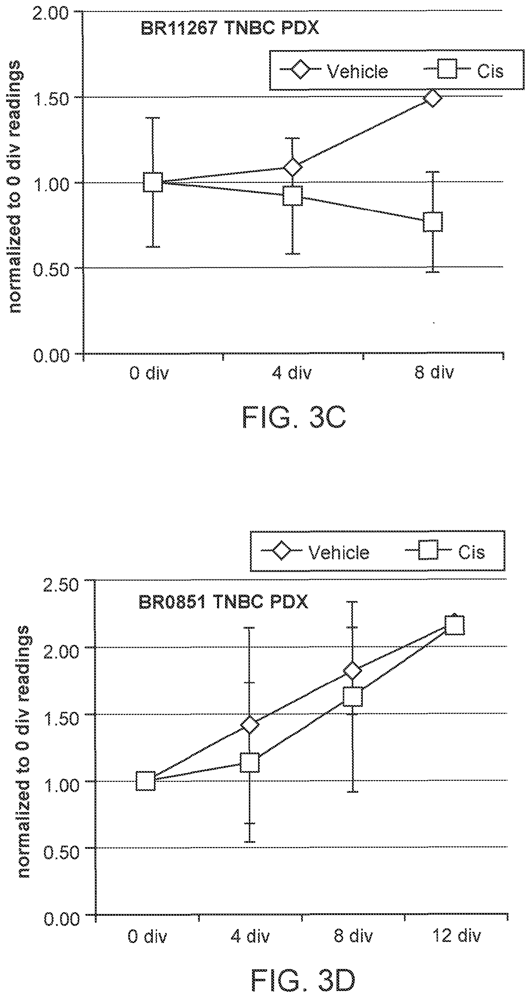

To determine whether *ex vivo* differences in drug response matches drug sensitivity in vivo, the response of two different TNBC PDX models for which in vivo drug response data exist (BR1126 and BR0851, JAX PDX Resource) was compared. *Ex vivo,* Cisplatin treatment resulted in a partial response in BR1126 TNBC PDX but not in BR0851 TNBC PDX slices (FIGs. 3C and 3D). This pattern is similar to in vivo treatment response where BR0851 showed no response while BR1126 showed partial response (FIGs. 3A and B). This correlation was evident even though treatment doses and scheduling were very different between the *in vivo* and *ex vivo* studies. These results suggest that *ex vivo* slice system can be used to predict relative in vivo drug sensitivity in a cost and time-efficient manner.

### Example 4: Unbiased pharmacological screening blind to genomics information using tumor slices

There are over 100 FDA approved oncology drugs, but the number of drugs used as standard of care for treatment of a particular tumor type in the clinic is only a small fraction of all available drugs. To perform an unbiased drug efficacy screen with a limited amount of tumor tissue from individual patients, the *ex vivo* slice culture system described herein was modified to perform high content drug screening. Approved Oncology Drugs Set VI, consisting of 119 FDA approved oncology drugs, was obtained from the National Cancer Institute (NCI). PDX tumor slices were obtained as described in Example 1 and 2mm diameter tumor slice punches were seeded into a 96 well plate and submerged in 200ul TSC medium. Viability was measured using the longitudinal Cell-Titer Blue (CTB) viability assay as described in Example 1 at days 1 and 3, and media was replaced following CTB measurements at day 1. Drugs were added at 100µM final concentration on day 1. Sample viability readings at day 3 (i.e., 2 days of drug exposure) were normalized to day 1 viability reading of each well and represented as fold change at day 3 compared to day 1 readings.

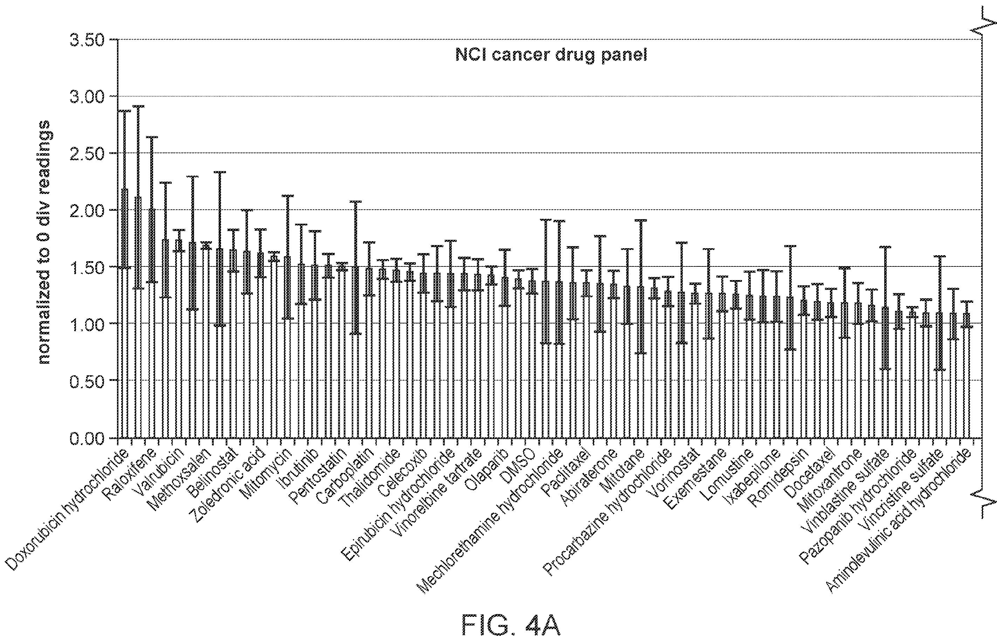

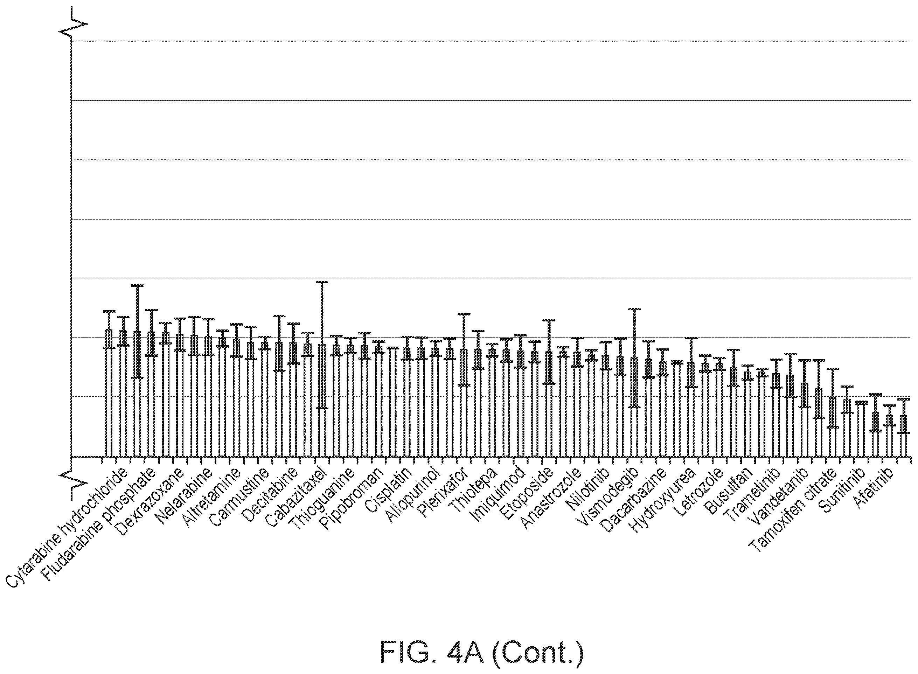

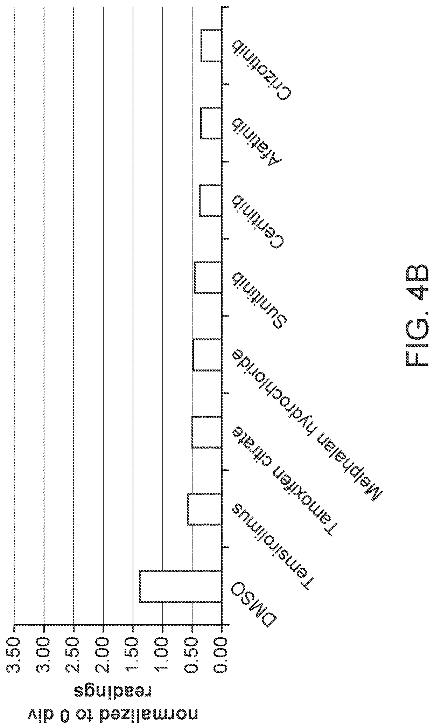

2mm punches were generated from two CN0458 colon PDX tumors so that each of the 119 FDA approved drugs on the NCI drug panel could be tested in triplicates. Cells were cultured for three days. Baseline viability of each slice was evaluated by taking CTB readings immediately after setting up the culture (0div). Each of the 119 drugs were added in duplicate wells after the baseline CTB reading. CTB reading was taken at 1 day post drug treatment to determine early response (1div) and again at three days post drug treatment (3div). To test reproducibility of the procedure, the same experiment was performed twice and highly consistent results were observed. FIG. 4A shows the average of the CTB measurements from these two experiments for each drug at 3 div (n=6 total), normalized to the baseline reading. Seven drugs reproducibly suppressed the viability >50% of the starting tissue. Two of these seven drugs have inhibitory activity against the same protein, ALK. None of the seven drugs are standard of care for mCRC patients currently, indicating that this method may be used to repurpose FDA approved oncology drugs. FIG. 4B shows the average CTB measurements for these seven drugs at higher magnification, normalized to the baseline reading.

### Example 5: Tumor slices accurately predict patient response

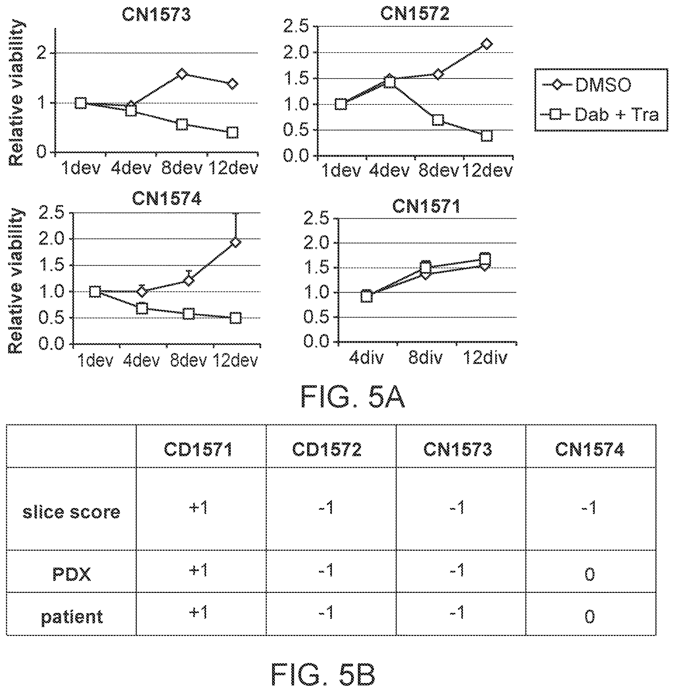

To validate the clinical utility of a tumor slice system, tissue samples from mCRC PDX models generated from four patients enrolled in a clinical trial (CN1571, CN1572, CN1573, and CN1574) were obtained, cultured, and evaluated using the methods described in Example 1. FIG. 5A shows viability analysis of tissue samples in response to dabrafenib (Dab) plus trametinib (Tra) combination therapy or DMSO control at the indicated days *ex vivo* (dev). Tumor slice response was compared to previously published data regarding patient response and in vivo PDX model response from the same four patients using modified RECIST criteria, outlined in Table 3, to correlate tumor slice response. The results of this comparison are shown in FIG. 5B. Tumor slice responses matched those of matching PDX and patients.

**Table 3**

| **Score** | **RECIST Criteria** | **Tumor slice viability** | **PDX tumor size** | **Patient tumor measurements** |
|---|---|---|---|---|
| **-2** | **CR** | <20% of day 0 | undetectable | undetectable |
| **-1** | **PR** | 21-70% of day 0 | >30% decrease | >30% decrease |
| **0** | **SD** | 71-120% of day 0 | 70-120 of baseline | 70-120% of baseline |
| **1** | **PD** | >121% of day 0 | >20% increase | >20% increase |

### Example 6: Response variability in cells from different locations of a tumor sample

Tumor tissue samples from two locations of the same patient tumor are obtained and cultured as described in Example 1. Each sample is treated with Cisplatin (Cis), Doxorubicin (Dox) or Paclitaxel (Taxol). Viability measurements are obtained at 0, 3, 5, 9, and 12 days in vitro. A significant difference between the viability measurements of each of the two samples is observed, demonstrating heterogeneity in drug response within the tumor tissue.

### Example 7: Tumor slices can be established and studied from biopsy needle cores.

Tumor tissue specimens from spontaneous MMTV-PyMT mouse breast tumor and CT26 mouse colorectal tumor were extracted using an 18-gauge core biopsy needle. Samples were precision cut into 250-350µM thick slices using a Vibratome VT1200S (Leica Microsystems). Each slice was cultured on thin, porous membranes (NucleoporeTM Track-Etched - Whatman^{®}) in 48-well culture dishes with 200µl of serum free tumorsphere culture medium (TSC medium: DMEM/F-12 (Hyclone^{®}), B27 supplement (Gibco^{®}), 100 U/mL penicillin (Gibco^{®}), and 100 µg/mL streptomycin (Gibco^{®})).

The MMTV-PyMT slices were treated with a control (DMSO) and chemotherapies: cyclophosphamide (CPP), doxorubicin (DOX), and a combination of cyclophosphamide, doxorubicin, and paclitaxel (CPP+DOX+TAXOL). Drugs were added to the MMTV-PyMT core slice cultures at the following final concentrations on Day 1: CPP at 20µM, DOX at 100 nM, and TAXOL at 1nM, and at each media change - days 1, 4, and 8.

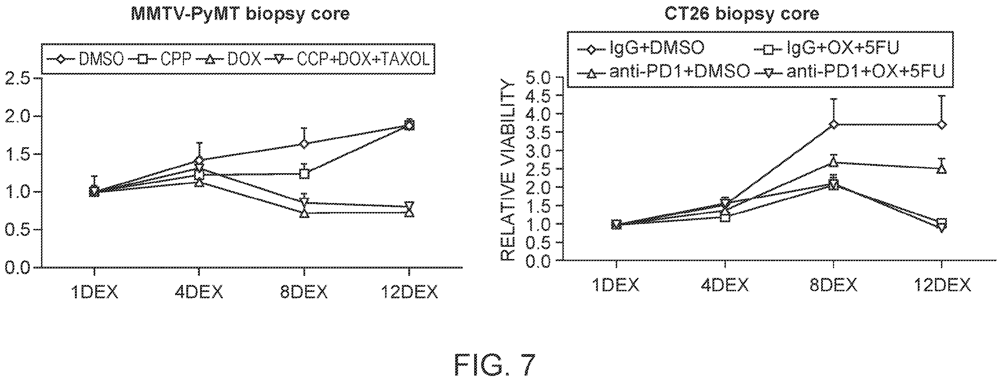

The CT26 core slices were then treated with a control, immunoglobulin G (IgG) and DMSO (IgG+DMSO), and chemo- and immunotherapy combinations, including IgG, Fluorouracil (5FU), and Oxaliplatin (Ox) (IgG+Ox+5FU); anti-PD1 and DMSO (anti-PD1+DMSO); and anti-PD1, 5FU and Oxaliplatin (anti-PD1+5FU+Ox). Drugs were added to the CT26 core slice cultures at the following final concentrations on Day 1: 5FU at 1µM, OX at 1µM, and anti-PD1 at 10 µg/ml.
Results were observed via viability measurement as described in Example 1. Representative results are shown in FIG. 7. Viability assay readings indicate that MMTV-PyMT tumor is sensitive to Dox and CCP+Dox+Taxol treatments and insensitive to CPP treatment, as demonstrated by increased viability readings in CPP treated slices and decreased viability readings in Dox and CCP+Dox+Taxol treated slices at day 12 compared to day 0. Additionally, viability assay readings indicate that the CT26 tumor is more sensitive to IgG+OX+5FU and anti-PD1+OX+5FU treatments and less sensitive to the anti-PD1+DMSO treatment.

### Example 8: Detecting emergence of therapy resistance using long-term tumor slice cultures.

Core needle biopsy samples of human metastatic colorectal cancer (mCRC) were obtained from patients in a clinical trial. Samples were precision cut into slices and cultured as described in Example 7. Samples were treated with targeted therapies - encorafenib (enco), a BRAFV600E inhibitor; cetuximab (cetux), an EGFR inhibitor; binimetinib (bini), an MEK inhibitor; and combinations thereof, including encorafenib and cetuximab (enco+cetux) and encorafenib, cetuximab, and binimetinib (enco+cetux+bini). The drugs were added to the tumor slice cultures at the following final concentrations on Day 1 and at each media change - Days 4, 8, 12, and 16: encorafenib at 100nM, cetuximab at 10 µg/mL, and binitetinib at 100nM.

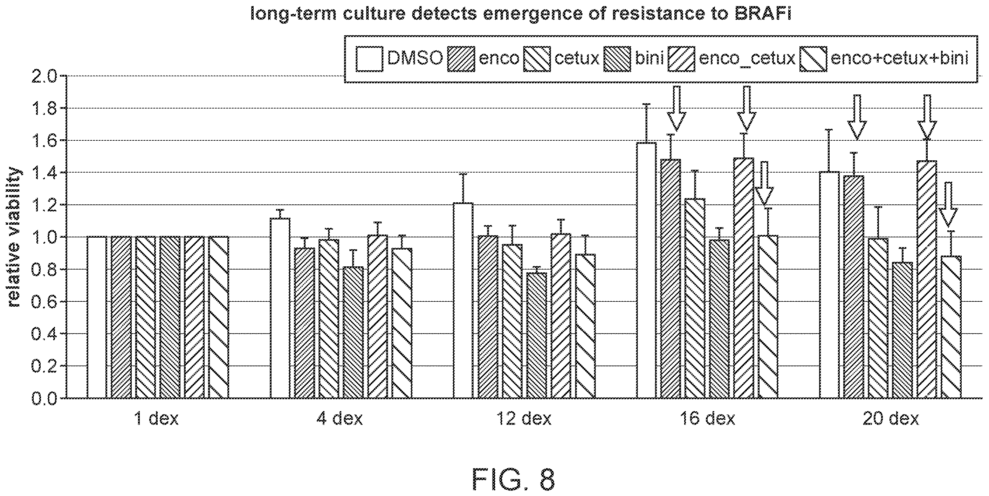

Results were observed via viability measurement as described in Example 1. Representative results are shown in FIG 8. FIG 8 illustrates that emergence of encorafenib resistance at 16 days in culture, which is suppressed by treatment with a downstream MEK inhibitor binitetinib (compare enco vs bini, and enco_cetux vs. enco+cetux+bini at days 16 and 20).

### Example 9: Long-term maintenance of resident immune cells.

A flow cytometry analysis of spontaneous MMTV-PyMT murine breast tumor slice cultures analyzed with anti-CD45, CD3, CD8 and CD4 antibodies was performed to assess the long-term maintenance of resident immune cells in tumor slices subject to the culturing conditions described herein. Tissue specimens from MMTV-PyMT mouse breast tumor were cultured for 4 days *ex vivo* with and without IL2. At 4 days *ex vivo,* the MMTV-PyMT tumor slices were dissociated using a cocktail containing a 9:1 ratio of accutase:collagenase + hyaloruronidase. Dissociated single cells were washed and resuspended in blocking antibody (antiCD16/32) and diluted in 2% BSA/PBS for 30 minutes on ice. Cells were then washed and resuspended in 45 µl of antibody cocktail containing anti-CD45, CD3, CD8, and CD4 antibodies (PE-CY7, PE, APC, FITC, BV650, and BV711) in Brilliant Violet staining buffer and incubated on ice for 30 minutes. After incubation, the cells were washed and stained with fixable viability dye for 30 minutes at room temperature. After staining, the cells were washed and fixed in ICfix solution and then analyzed on a LSRII flow cytometer.

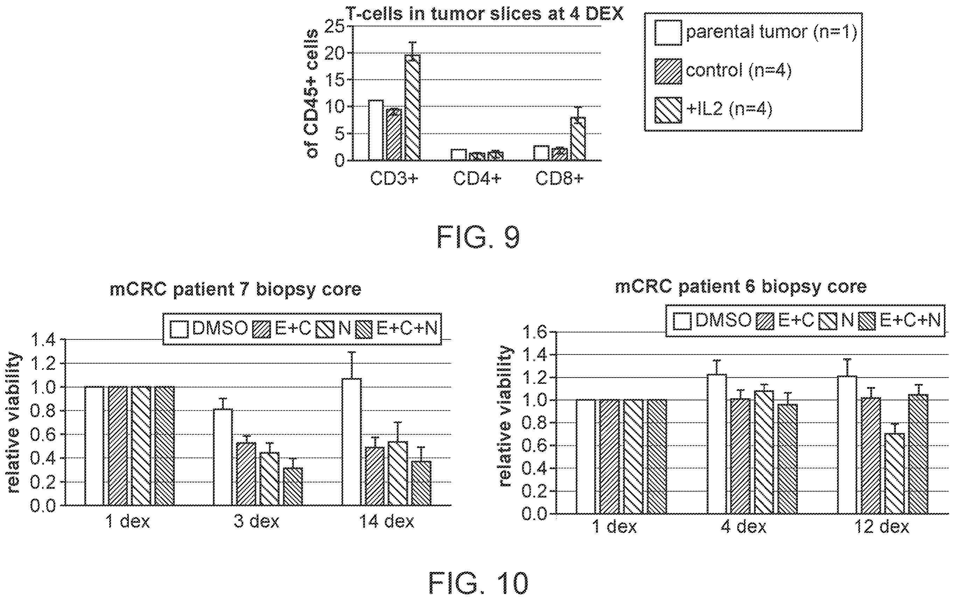

The results are shown in FIG 9A. CD3+, CD4+, and CD8+ cells in the slice cultures were maintained at a frequency close to parental tumor frequency (without the addition of IL2), supporting that the tumor slices are representative of the primary tumor tissue with respect to the immune cell microenvironment. With the addition of IL2, immune cell frequency was increased with respect to parental tumor frequency.

### Example 10: Measuring patient-specific responses to immunotherapies.

Core needle biopsy was used to obtain samples of human metastatic colorectal cancer (mCRC) biopsy cores from two patients in a clinical trial. Samples were precision cut into slices and cultured as described in Example 7. Samples from each patient were then treated with a control (DMSO) and encorafenib and cetuximab (E+C), the immune checkpoint inhibitor nivolumab (N), and a combination of encorafenib, cetuximab, and nivolumab (E+C+N). The drugs were added to the tumor slice cultures at the following final concentrations on Day 1, 4, 8, 12, and 16: encorafenib at 100nM, cetuximab at 10 µg/mL, and binimetinib at 100nM and nivolumab at 10 µg/mL. The samples were cultured for 14 days.

Results were observed via viability measurement as described in Example 1. The results are shown in FIG 10. The two mCRC patient samples showed differential sensitivity to the checkpoint inhibitor nivolumab alone and in combination with encorafenib and cetuximab (E+C+N) at early and late time points.

While preferred embodiments of the present disclosure have been shown and described herein, it will be obvious to those skilled in the art that such embodiments are provided by way of example only. Numerous variations, changes, and substitutions will now occur to those skilled in the art without departing from the disclosure. It should be understood that various alternatives to the embodiments of the disclosure described herein may be employed in practicing the disclosure. It is intended that the following claims define the scope of the disclosure and that methods and structures within the scope of these claims and their equivalents be covered thereby.

### EXAMPLES

1. A method of assessing a candidate molecule, comprising:
   (a) administering the candidate molecule to a cell sample of a tumor tissue derived from a subject;
   (b) culturing the cell sample in the presence of the candidate molecule for at least 1 day; and
   (c) assessing a response of the cell sample to the candidate molecule.
2. The method of example 1, wherein, in (b), the cell sample is cultured for at least 2 days, at least 3 days, at least 4 days, at least 5 days, 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, or at least 20 days.
3. The method of example 2, wherein, in (b), the cell sample is cultured for 12 days.
4. The method of example 1, further comprising (d) administering an additional candidate molecule to the cell sample and culturing the cell sample for at least 24 hours.
5. The method of example 4, wherein, in (d), the cell sample is cultured for at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, or at least 20 days.
6. The method of example 5, wherein, in (d), the cell sample is cultured for 12 days.
7. The method of example 4, further comprising (e) assessing a response of the cell sample to the additional candidate molecule.
8. The method of example 1, further comprising (d) administering a second dose of the candidate molecule to the cell sample and culturing the cell sample for at least 24 hours.
9. The method of example 8, wherein, in (d), the cell sample is cultured for at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, or at least 20 days.
10. The method of example 9, wherein, in (d), the cell sample is cultured for 12 days.
11. The method of example 8, further comprising (e) assessing a response of the cell sample to the second dose of the candidate molecule.
12. The method of example 1, wherein the cell sample comprises a plurality of cells.
13. The method of example 1, wherein the cell sample is an intact tissue.
14. The method of example 1, wherein the cell sample is an organoid.
15. The method of example 1, wherein the cell sample is isolated from the tumor tissue.
16. The method of example 1, wherein the cell sample comprises a cancer cell.
17. The method of example 1, wherein the cell sample comprises a cell from a microenvironment of the tumor tissue.
18. The method of example 17, wherein the cell from the microenvironment of the tumor tissue is an immune cell, a fibroblast, an endothelial cell, a pericyte, an adipocyte, a mesenchymal stem cell, or a bone marrow-derived cell.
19. The method of example 18, wherein the immune cell is a lymphoid cell or a myeloid cell.
20. The method of example 19, wherein the lymphoid cell is a T cell, a B cell, a natural killer cell, a dendritic cell.
21. The method of example 19, wherein the myeloid cell is a basophil, an eosinophil, a mast cell, a neutrophil, a monocyte, an erythrocyte, a macrophage, a myeloid derived suppressor cell, or a dendritic cell.
22. The method of example 1, wherein the cell sample is cultured in serum-free conditions.
23. The method of example 1, wherein the cell sample is cultured at a liquid-air interface.
24. The method of example 1, wherein the cell sample is submerged in cell culture media.
25. The method of example 1, wherein the response is a viability response, a gene expression response, a protein expression response, a protein modification response, a cell signaling response, a morphology response, or a metabolic response.
26. The method of example 1, wherein assessing a viability response comprises measuring a metabolic product from the cell sample.
27. The method of example 26, wherein measuring the metabolic product from the cell sample comprises isolating the metabolic product from cell culture media and measuring the metabolic product in the absence of the cell sample.
28. The method of example 26, wherein the metabolic product from the cell sample is a product of a reduction reaction.
29. The method of example 26, wherein the metabolic product from the cell sample is derived from resazurin.
30. The method of example 29, wherein the metabolic product from the cell sample is resorufin.
31. The method of example 26, wherein the metabolic product from the cell sample is derived from a tetrazole.
32. The method of example 31, wherein the tetrazole is selected from the group consisting of 3-(4,5-dimethylthiazol-2-yl)-2,5-diphenyltetrazolium (MTT) or a salt thereof, 2,3-bis-(2-methoxy-4-nitro-5-sulfophenyl)-2H-tetrazolium-5-carboxanilide (XTT) or a salt thereof, and 3-(4,5-dimethylthiazol-2-yl)-5-(3-carboxymethoxyphenyl)-2-(4-sulfophenyl)-2H-tetrazolium (MTS) or a salt thereof.
33. The method of example 31, wherein the metabolic product from the cell sample is a formazan.
34. The method of example 1, wherein the cell sample is from a patient-derived xenograft.
35. The method of example 1, wherein the cell sample is from a primary tumor sample.
36. The method of example 35, wherein the primary tumor sample is a surgical sample from a subject.
37. The method of example 35, wherein the primary tumor sample is a biopsy sample obtained from a patient.
38. The method of example 37, wherein the biopsy sample is obtained by a core needle biopsy.
39. The method of example 1, further comprising recommending treatment of a subject with the candidate molecule.
40. The method of example 1, further comprising recommending not treating a subject with the candidate molecule.
41. The method of example 1, wherein the candidate molecule is selected from the group consisting of a biological molecule and a chemical molecule.
42. The method of example 41, wherein the biological molecule is selected from the group consisting of a recombinant protein, an enzyme, an antibody, a growth factor, a receptor, and any portion thereof.
43. The method of example 1, wherein (a) further comprises providing one or more additional candidate molecules, wherein (c) comprises assessing the response of the cell sample to the candidate molecule and the one or more additional candidate molecules.
44. The method of example 43, wherein the one or more additional candidate molecules are provided simultaneously with the candidate molecule.
45. The method of example 43, wherein the candidate molecule and the one or more additional candidate molecules are provided sequentially.
46. The method of example 1, further comprising:
   (d) administering the candidate molecule to an additional cell sample of the tumor tissue derived from the subject;
   (e) culturing the additional cell sample in the presence of the candidate molecule for at least 5 days;
   (f) assessing a response of the additional cell sample to the candidate molecule; and
   (g) comparing the response of the additional cell sample to the response of the cell sample.
47. The method of example 466, wherein the cell sample is derived from a first location of the tumor tissue and the additional cell sample is derived from a second location of the tumor tissue.
48. The method of example 46, wherein the cell sample and the additional cell sample are obtained from the tumor tissue at different times.
49. The method of example 486, wherein the different times are at least 1, 2, 3, 4, 5, 10, 15, or 20 days apart.
50. The method of example 46, wherein the cell sample and the additional cell sample are obtained from the tumor tissue at substantially the same time.
51. The method of example 1, further comprising performing a biological assay on the cell sample.
52. The method of example 51, wherein performing the biological assay on the cell sample comprises fixing the cell sample.
53. The method of example 51, wherein performing the biological assay on the cell sample comprises permeabilizing the cell sample.
54. The method of example 51, wherein performing the biological assay on the cell sample comprises lysing the cell sample.
55. The method of example 51, wherein the biological assay is selected from the group consisting of flow cytometry, nucleic acid sequencing, polymerase chain reaction, histological analysis, immunohistochemistry, immunofluorescence, enzyme-linked immunosorbent assay (ELISA) analysis, mass spectrometry, and any combination thereof.
56. A method of assessing a candidate molecule, comprising:
   (a) administering the candidate molecule to a cell sample of a tumor tissue derived from a subject;
   (b) culturing the cell sample of the tumor tissue for at least 1 day;
   (c) assessing a response of the cell sample of the tumor tissue to the candidate molecule; and
   (d) repeating (a)-(c).
57. The method of example 56, wherein, in (b), the cell sample is cultured for at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, or at least 20 days.
58. The method of example 57, wherein, in (b), the cell sample is cultured for 4 days.
59. The method of example 56, wherein (d) comprises repeating (a)-(c) at least 1 time, at least 2 times, at least 3 times, at least 4 times, at least 5 times, or at least 6 times.
60. The method of example 59, wherein (d) comprises repeating (a)-(d) 3 times.
61. The method of example 59, wherein the cell sample comprises a plurality of cells.
62. The method of example 59, wherein the cell sample is an intact tissue.
63. The method of example 59, wherein the cell sample is an organoid.
64. The method of example 59, wherein the cell sample is isolated from the tumor tissue.
65. The method of example 59, wherein the cell sample comprises a cancer cell.
66. The method of example 59, wherein the cell sample comprises a cell from a microenvironment of the tumor tissue.
67. The method of example 66, wherein the cell from the microenvironment of the tumor tissue is an immune cell, a fibroblast, an endothelial cell, a pericyte, an adipocyte, a mesenchymal stem cell, or a bone marrow-derived cell.
68. The method of example 67, wherein the immune cell is a lymphoid cell or a myeloid cell.
69. The method of example 68, wherein the lymphoid cell is a T cell, a B cell, a natural killer cell, a dendritic cell.
70. The method of example 68, wherein the myeloid cell is a basophil, an eosinophil, a mast cell, a neutrophil, a monocyte, an erythrocyte, a macrophage, a myeloid derived suppressor cell, or a dendritic cell.
71. The method of example 56, wherein the cell sample is cultured in serum-free conditions.
72. The method of example 56, wherein the cell sample is cultured at a liquid-air interface.
73. The method of example 56, wherein the cell sample is submerged in cell culture media.
74. The method of example 56, wherein the response is a viability response, a gene expression response, a protein expression response, a protein modification response, a cell signaling response, a morphology response, or a metabolic response.
75. The method of example 56, wherein the assessing the response comprises measuring a metabolic product from the cell sample.
76. The method of example 75, wherein measuring the metabolic product from the cell sample comprises isolating the metabolic product from cell culture media and measuring the metabolic product in the absence of the cell sample.
77. The method of example 75, wherein the metabolic product from the cell sample is a product of a reduction reaction.
78. The method of example 75, wherein the metabolic product from the cell sample is derived from resazurin.
79. The method of example 78, wherein the metabolic product from the cell sample is resorufin.
80. The method of example 77, wherein the metabolic product from the cell sample is derived from a tetrazole.
81. The method of example 80, wherein the tetrazole is selected from the group consisting of 3-(4,5-dimethylthiazol-2-yl)-2,5-diphenyltetrazolium (MTT) or a salt thereof, 2,3-bis-(2-methoxy-4-nitro-5-sulfophenyl)-2H-tetrazolium-5-carboxanilide (XTT) or a salt thereof, and 3-(4,5-dimethylthiazol-2-yl)-5-(3-carboxymethoxyphenyl)-2-(4-sulfophenyl)-2H-tetrazolium (MTS) or a salt thereof.
82. The method of example 80, wherein the metabolic product from the cell sample is a formazan.
83. The method of example 56, wherein the cell sample is from a patient-derived xenograft.
84. The method of example 56, wherein the cell sample is from a primary tumor sample.
85. The method of example 84, wherein the primary tumor sample is a surgical sample obtained from a patient.
86. The method of example 84, wherein the primary tumor sample is a biopsy sample obtained from a patient.
87. The method of example 86, wherein the biopsy sample is obtained by a core needle biopsy.
88. The method of example 56, further comprising recommending treatment of a subject with the candidate molecule.
89. The method of example 56, further comprising recommending not treating a subject with the candidate molecule.
90. The method of example 56, wherein the candidate molecule is selected from the group consisting of a biological molecule and a chemical molecule.
91. The method of example 56, wherein (a) further comprises providing one or more additional candidate molecules, wherein (c) comprises assessing the response of the cell sample to the candidate molecule and the one or more additional candidate molecules.
92. The method of example 91, wherein the one or more additional candidate molecules are provided simultaneously with the candidate molecule.
93. The method of example 91, wherein the candidate molecule and the one or more additional candidate molecules are provided sequentially.
94. The method of example 56, further comprising:
   (e) administering the candidate molecule to an additional cell sample of the tumor tissue derived from the subject;
   (f) culturing the additional cell sample of the tumor tissue for at least 1 day;
   (g) assessing a response of the additional cell sample of the tumor tissue to the candidate molecule;
   (h) repeating (e) - (g); and
   (i) comparing the response of the additional cell sample to the response of the cell sample
95. The method of example 94, wherein the cell sample is derived from a first location of the tumor tissue and the additional cell sample is derived from a second location of the tumor tissue.
96. The method of example 94, wherein the cell sample and the additional cell sample are obtained from the tumor tissue at different times.
97. The method of example 96, wherein the different times are at least 1, 2, 3, 4, or 5 days apart.
98. The method of example 95, wherein the cell sample and the additional cell sample are obtained from the tumor tissue at substantially the same time.
99. The method of example 56, further comprising performing a biological assay on the cell sample.
100. The method of example 99, wherein performing the biological assay on the cell sample comprises fixing the cell sample.
101. The method of example 99, wherein performing the biological assay on the cell sample comprises permeabilizing the cell sample.
102. The method of example 99, wherein performing the biological assay on the cell sample comprises lysing the cell sample.
103. The method of example 99, wherein the biological assay is selected from the group consisting of flow cytometry, nucleic acid sequencing, polymerase chain reaction, histological analysis, immunohistochemistry, immunofluorescence, enzyme-linked immunosorbent assay (ELISA) analysis, mass spectrometry, and any combination thereof.
104. A method of assessing cellular viability, comprising:
   (a) culturing a cell sample of a tumor in cell culture media;
   (b) isolating a metabolic product from the cell culture media; and
   (c) measuring the metabolic product in the absence of the cell sample, thereby assessing cellular viability of the cell sample.
105. The method of example 104, wherein the cell sample is from a cell line.
106. The method of example 104, wherein the cell sample comprises a plurality of cells.
107. The method of example 104, wherein the cell sample is an intact tissue.
108. The method of example 104, wherein the cell sample is an organoid.
109. The method of example 104, wherein the cell sample is isolated from a tumor.
110. The method of example 104, wherein the cell sample comprises a cancer cell.
111. The method of example 104, wherein the cell sample comprises a cell from a microenvironment of a tumor tissue.
112. The method of example 111, wherein the cell from the microenvironment of the tumor tissue is an immune cell, a fibroblast, an endothelial cell, a pericyte, an adipocyte, a mesenchymal stem cell, or a bone marrow-derived cell.
113. The method of example 112, wherein the immune cell is a lymphoid cell or a myeloid cell.
114. The method of example 113, wherein the lymphoid cell is a T cell, a B cell, a natural killer cell, a dendritic cell.
115. The method of example 113, wherein the myeloid cell is a basophil, an eosinophil, a mast cell, a neutrophil, a monocyte, an erythrocyte, a macrophage, a myeloid derived suppressor cell, or a dendritic cell.
116. The method of example 104, wherein the cell sample is cultured in serum-free conditions.
117. The method of example 104, wherein the cell sample is cultured at a liquid-air interface.
118. The method of example 104, wherein the cell sample is submerged in cell culture media.
119. The method of example 104, wherein measuring the metabolic product comprises a fluorescence measurement or a chemiluminescence measurement.
120. The method of example 104, wherein the cell sample is cultured for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, or at least 20 days.
121. The method of example 120, wherein the cell sample is cultured for 12 days.
122. The method of example 104, wherein the metabolic product from the cell sample is a product of a reduction reaction.
123. The method of example 104, wherein the metabolic product from the cell sample is derived from resazurin.
124. The method of example 123, wherein the metabolic product from the cell sample is resorufin.
125. The method of example 104, wherein the metabolic product from the cell sample is derived from a tetrazole.
126. The method of example 125, wherein the tetrazole is selected from the group consisting of 3-(4,5-dimethylthiazol-2-yl)-2,5-diphenyltetrazolium (MTT) or a salt thereof, 2,3-bis-(2-methoxy-4-nitro-5-sulfophenyl)-2H-tetrazolium-5-carboxanilide (XTT) or a salt thereof, and 3-(4,5-dimethylthiazol-2-yl)-5-(3-carboxymethoxyphenyl)-2-(4-sulfophenyl)-2H-tetrazolium (MTS) or a salt thereof.
127. The method of example 125, wherein the metabolic product from the cell sample is a formazan.
128. The method of example 104, further comprising administering a candidate molecule to the cell sample.
129. The method of example 104, wherein the cell sample is from a patient-derived xenograft.
130. The method of example 104, wherein the cell sample is from a primary tumor sample.
131. The method of example 130, wherein the primary tumor sample is a surgical sample.
132. The method of example 130, wherein the primary tumor sample is a biopsy sample obtained from a patient.
133. The method of example 132, wherein the biopsy sample is obtained by a core needle biopsy.
134. The method of example 104, further comprising recommending treatment of a subject with the candidate molecule.
135. The method of example 104, further comprising recommending not treating a subject with the candidate molecule.
136. The method of example 104, further comprising:
   (d) culturing an additional cell sample in additional cell culture media;
   (e) isolating a metabolic product from the additional cell culture media;
   (f) measuring the metabolic product from the additional cell culture media in the absence of the additional cell sample, thereby assessing cellular viability of the additional cell sample; and
   (g) comparing the cellular viability of the additional cell sample to the cellular viability of the cell sample.
137. The method of example 136, wherein the cell sample is derived from a first location of a tumor tissue and the additional cell sample is derived from a second location of the tumor tissue.
138. The method of example 136, wherein the cell sample and the additional cell sample are obtained from the tumor tissue at different times.
139. The method of example 138, wherein the different times are at least 1, 2, 3, 4, 5, 10, 15, or 20 days apart.
140. The method of example 136, wherein the cell sample and the additional cell sample are obtained from the tumor tissue at substantially the same time.
141. The method of example 104, wherein the tumor tissue is a tissue sample derived from a subject.
142. A method of assessing a radiation dose, comprising:
   (a) administering the radiation dose to a cell sample of a tumor tissue derived from a subject;
   (b) culturing the cell sample for at least 1 day; and
   (c) assessing a response of the cell sample to the radiation dose.
143. A method of assessing a tumor treating field, comprising:
   (a) exposing a cell sample of a tumor tissue derived from a subject to a tumor treating field;
   (b) culturing the cell sample for at least 1 day; and
   (c) assessing a response of the cell sample to the tumor treating field.
144. A method of assessing a radiation dose, comprising:
   (a) administering the radiation dose to a cell sample of a tumor tissue derived from a subject;
   (b) culturing the cell sample of the tumor tissue for at least 1 day;
   (c) assessing a response of the cell sample of the tumor tissue to the radiation dose; and
   (d) repeating (a)-(c).
145. A method of assessing a tumor treating field, comprising:
   (a) exposing a cell sample of a tumor tissue derived from a subject to a tumor treating field;
   (b) culturing the cell sample of the tumor tissue for at least 1 day;
   (c) assessing a response of the cell sample of the tumor tissue to the tumor treating field; and
   (d) repeating (a)-(c).
146. A method of assessing a candidate molecule, comprising:
   (a) administering the candidate molecule to a cell sample of a tumor tissue derived from a subject;
   (b) culturing the cell sample in the presence of the candidate molecule for at least 12 days; and
   (c) assessing a response of the cell sample to the candidate molecule.
147. A method of assessing a candidate molecule, comprising:
   (a) administering the candidate molecule to a cell sample of a tumor tissue derived from a subject;
   (b) culturing the cell sample of the tumor tissue for at least 12 days;
   (c) assessing a response of the cell sample of the tumor tissue to the candidate molecule; and
   (d) repeating (a)-(c).
148. A method of assessing cellular viability, comprising:
   (a) culturing a cell sample of a tumor in cell culture media for at least 4 days;
   (b) isolating a metabolic product from the cell culture media; and
   (c) measuring the metabolic product in the absence of the cell sample, thereby assessing cellular viability of the cell sample.
149. A method of assessing a candidate molecule, comprising:
   (a) administering the candidate molecule to a cell sample of a tumor tissue derived from a subject;
   (b) culturing the cell sample of the tumor tissue for at least 4 days;
   (c) assessing a response of the cell sample of the tumor tissue to the candidate molecule; and
   (d) repeating (a)-(c) at least 3 times.
150. A method of assessing a radiation dose, comprising:
   (a) administering the radiation dose to a cell sample of a tumor tissue derived from a subject;
   (b) culturing the cell sample for at least 12 days; and
   (c) assessing a response of the cell sample to the radiation dose.
151. A method of assessing a tumor treating field, comprising:
   (a) exposing a cell sample of a tumor tissue derived from a subject to a tumor treating field;
   (b) culturing the cell sample for at least 12 days; and
   (c) assessing a response of the cell sample to the tumor treating field.
152. A method of assessing a radiation dose, comprising:
   (a) administering the radiation dose to a cell sample of a tumor tissue derived from a subject;
   (b) culturing the cell sample of the tumor tissue for at least 12 days;
   (c) assessing a response of the cell sample of the tumor tissue to the radiation dose; and
   (d) repeating (a)-(c).
153. A method of assessing a tumor treating field, comprising:
   (a) exposing a cell sample of a tumor tissue derived from a subject to a tumor treating field;
   (b) culturing the cell sample of the tumor tissue for at least 12 days;
   (c) assessing a response of the cell sample of the tumor tissue to the tumor treating field; and
   (d) repeating (a)-(c).

## Claims

1. A method of assessing cellular viability, comprising:
(a) culturing a cell sample of a tumor in cell culture media;
(b) isolating a metabolic product from the cell culture media; and
(c) measuring the metabolic product in the absence of the cell sample, thereby assessing cellular viability of the cell sample.

2. The method of claim 1, wherein the cell sample is from a cell line,
comprises a plurality of cells,
is an intact tissue,
sample is an organoid,
is isolated from a tumor,or/and
comprises a cancer cell.

3. The method of claim 104, wherein the cell sample comprises a cell from a microenvironment of a tumor tissue.

4. The method of claim 3, wherein the cell from the microenvironment of the tumor tissue is an immune cell, a fibroblast, an endothelial cell, a pericyte, an adipocyte, a mesenchymal stem cell, or a bone marrow-derived cell.

5. The method of claim 3, wherein the immune cell is a lymphoid cell or a myeloid cell.

6. The method of claim 5, wherein the lymphoid cell is a T cell, a B cell, a natural killer cell, a dendritic cell, or
wherein the myeloid cell is a basophil, an eosinophil, a mast cell, a neutrophil, a monocyte, an erythrocyte, a macrophage, a myeloid derived suppressor cell, or a dendritic cell.

7. The method of claim 1, wherein the cell sample is cultured in serum-free conditions, at a liquid-air interface,
wherein the cell sample is submerged in cell culture media,

8. wherein measuring the metabolic product comprises a fluorescence measurement or a chemiluminescence measurement,
wherein the cell sample is cultured for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, or at least 20 days, or/and
wherein the metabolic product from the cell sample is a product of a reduction reaction.

9. The method of claim 1, wherein the metabolic product from the cell sample is derived from resazurin,
wherein optionally the metabolic product from the cell sample is resorufin.

10. The method of claim 1, wherein the metabolic product from the cell sample is derived from a tetrazole,
wherein optionally the tetrazole is selected from the group consisting of a formazan, 3-(4,5-dimethylthiazol-2-yl)-2,5-diphenyltetrazolium (MTT) or a salt thereof, 2,3-bis-(2-methoxy-4-nitro-5-sulfophenyl)-2H-tetrazolium-5-carboxanilide (XTT) or a salt thereof, and 3-(4,5-dimethylthiazol-2-yl)-5-(3-carboxymethoxyphenyl)-2-(4-sulfophenyl)-2H-tetrazolium (MTS) or a salt thereof.

11. The method of claim 1, further comprising administering a candidate molecule to the cell sample, or/and
wherein the cell sample is from a patient-derived xenograft.

12. The method of claim 1, wherein the cell sample is from a primary tumor sample.

13. The method of claim 1, further comprising:
(d) culturing an additional cell sample in additional cell culture media;
(e) isolating a metabolic product from the additional cell culture media;
(f) measuring the metabolic product from the additional cell culture media in the absence of the additional cell sample, thereby assessing cellular viability of the additional cell sample; and
(g) comparing the cellular viability of the additional cell sample to the cellular viability of the cell sample.

14. The method of claim 13, wherein the cell sample is derived from a first location of a tumor tissue and the additional cell sample is derived from a second location of the tumor tissue or
wherein the cell sample and the additional cell sample are obtained from the tumor tissue at different times,
wherein the different times are at least 1, 2, 3, 4, 5, 10, 15, or 20 days apart.

15. The method of claim 13, wherein the cell sample and the additional cell sample are obtained from the tumor tissue at substantially the same time.
